# EUROPÄISCHE PATENTSCHRIFT

(11) **EP 3 029 056 B1**
(45) Veröffentlichungstag und Bekanntmachung des Hinweises auf die Patenterteilung: **07.11.2018**
(21) Anmeldenummer: 15173827.5
(22) Anmeldetag: 04.12.2014
(51) Int. Cl.: C07F 9/6574, C07B 41/06, C07C 45/50

(54) **BISPHOSPHITE DIE EINEN UNSYMMETRISCHEN BIARYL-ZENTRAL-BAUSTEIN AUFWEISEN**
BIS-PHOSPHITES WITH AN ASYMMETRIC BIARYL CENTRAL COMPONENT
BISPHOSPHITES PRESENTANT UN COMPOSANT CENTRAL EN BI-ARYLE ASYMETRIQUE

(43) Veröffentlichungstag der Anmeldung: 08.06.2016
(62) Teilanmeldung aus: 14196178.9
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Börner, Armin, 18059 Rostock (DE); Selent, Detlef, 18059 Rostock (DE)

(56) Entgegenhaltungen:
- EP-B1- 0 900 187
- WO-A1-2008/071508
- DETLEF SELENT ET AL: "A New Diphosphite Promoting Highly Regioselective Rhodium-Catalyzed Hydroformylation", ORGANOMETALLICS, Bd. 30, Nr. 17, 12. September 2011 (2011-09-12), Seiten 4509-4514, XP055186800, ISSN: 0276-7333, DOI: 10.1021/om2000508

## Beschreibung

Die Erfindung betrifft Bisphosphite, die einen unsymmetrischen Biaryl-Zentral-Baustein aufweisen. Des Weiteren deren Verwendung als Liganden in der Hydroformylierung.

Ein Bisphosphit weist einen Zentral-Baustein, das so genannte Rückgrat, und zwei Flügel-Bausteine auf, welche mit dem Zentral-Baustein über das P-Atom verbunden sind.

Die erfindungsgemäßen Biaryl-Zentral-Bausteine weisen beispielsweise eine Phenyl-Phenyl-Einheit, oder eine Naphthyl-Phenyl-Einheit auf.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (=n) zu verzweigtem (=iso) Aldehyd)) erreicht. In WO 95/30680 werden zweizähnige Phosphinliganden und ihr Einsatz in der Katalyse, unter anderem auch in Hydroformylierungsreaktionen, offenbart.

In DE 10 2006 058 682 A1 werden Bisphosphite offenbart, welche einen symmetrischen Zentral-Baustein (Y) aufweisen. Dieser kann aus den in DE 10 2006 058 682 A1 Absatz [0022] offenbarten Formeln IIa bis IId oder III ausgewählt sein.

In EP 0 900 187 B1 wird ein Verfahren zur Herstellung Hydroxyaldehyde beschreiben. Hierbei wird in einem ersten Schritt mittels reduzierender Hydroformylierung aus einem Alkadien ein ungesättigter Alkohol hergestellt. Der Alkohol wird dann in einem zweiten Schritt mittels Hydroformylierung zum Hydroxyaldehyd umgesetzt.

In WO 2008/071508 A1 wird ein Bisphosphit beschrieben, sowie ein Verfahren zu dessen Herstellung.

In DETLEF SELENT ET AL, "A New Diphosphite Promoting Highly Regioselective Rhodium-Catalyzed Hydroformylation", ORGANOMETALLICS, (20110912), Bd. 30, Nr. 17, Seiten 4509 -4514 wird ein Rhodium-Katalysator beschrieben, welcher ein hohes Maß an Regioselektivität bei der Hydroformylierung liefert.

Obgleich eine Vielzahl von Liganden und ihre Verwendung in der Rhodium-katalysierten Hydroformylierung bekannt sind, ist es wünschenswert, neue Liganden mit verbesserten Eigenschaften zu entwickeln.

Der Erfindung lag die Aufgabe zugrunde, Bisphosphite bereitzustellen, welche gegenüber den bekannten Bisphosphiten vorteilhafte Eigenschaften in der Hydroformylierung aufweisen. Insbesondere bestand die Aufgabe darin, neue Liganden bereitzustellen, die neben einer guten Ausbeute auch noch eine hohe n-Selektivität der korrespondierende Aldehyde bei der Umsetzung von endständigen Olefinen generieren und die ebenfalls bei der Hydroformylierung von innenständigen Olefinen zufriedenstellende n/i-Selektivitäten aufweisen. Es soll also neben einer guten Ausbeute auch noch zusätzlich eine gute Selektivität erzielt werden.

Die Aufgabe wird gelöst durch eine Verbindung nach Anspruch 1.

Verbindung, welche die allgemeine Struktur **II** aufweist: 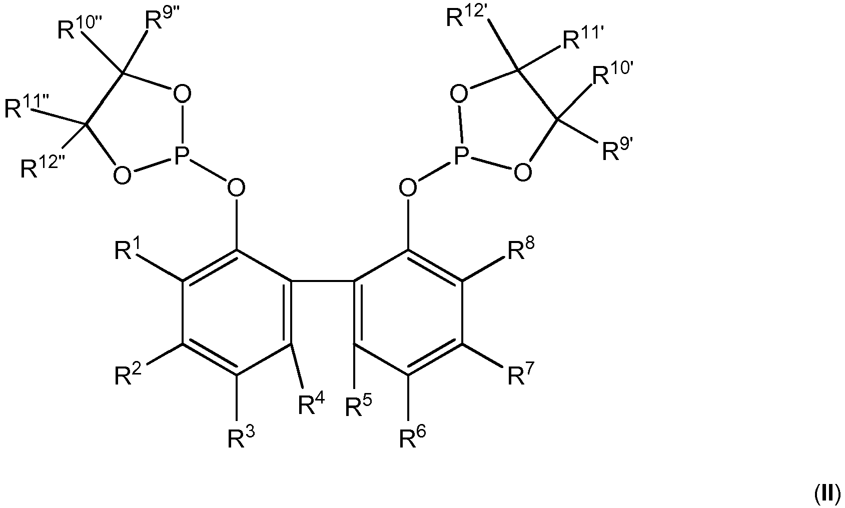 wobei
R¹, R², R³, R⁴, R⁵, R⁶, R⁷, R⁸ ausgewählt sind aus:
   -H, -(C₁-C₁₂)-Alkyl, -O-(C₁-C₁₂)-Alkyl, -O-(C₆-C₂₆)-Aryl, -(C₆-C₂₆)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹', R¹⁰', R¹¹', R¹²', R⁹", R¹⁰", R¹¹", R¹²" ausgewählt sind aus:
   -H, -(C₆-C₂₆)-Aryl;
und die beiden Reste mindestens eines der vier folgenden Restepaare nicht für den gleichen Rest stehen: R¹ und R⁸, R² und R⁷, R³ und R⁶, R⁴ und R⁵;
wobei die genannten Alkyl- und Arylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₆)-Aryl, Fluor, Chlor, Cyano, Formyl, Acyl oder Alkoxycarbonyl;
   substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₆)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na, -NO₂, -CN, -NH₂, -N[(C₁-C₁₂)-Alkyl]₂.

Durch das Merkmal "und die beiden Reste mindestens eines der vier folgenden Restepaare nicht für den gleichen Rest stehen: R¹ und R⁸, R² und R⁷, R³ und R⁶, R⁴ und R⁵" wird zum Ausdruck gebracht, dass es sich hierbei um ein unsymmetrisches Biaryl handelt. Die beiden Aromaten lassen sich nicht durch eine zwischen ihnen liegende Spiegelebene aufeinander spiegeln.

Zugelassen sind folgende Restepaarungen, wie beispielsweise:
R¹ ungleich R⁸, R² gleich R⁷, R³ gleich R⁶, R⁴ gleich R⁵;
R¹ gleich R⁸, R² gleich R⁷, R³ ungleich R⁶, R⁴ gleich R⁵.

Ober aber auch Restepaarungen bei denen mehr als nur ein Paar ungleich ist, wie beispielsweise:
R¹ ungleich R⁸, R² gleich R⁷, R³ ungleich R⁶, R⁴ gleich R⁵;
R¹ ungleich R⁸, R² ungleich R⁷, R³ ungleich R⁶, R⁴ gleich R⁵.

Ausgeschlossen wird lediglich der Fall, bei dem alle vier Restepaare jeweils paarweise für den gleichen Rest stehen:
R¹ gleich R⁸, R² gleich R⁷, R³ gleich R⁶, R⁴ gleich R⁵.
Hierbei würde es sich um ein symmetrisches Biaryl handeln.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₆)-Aryl, -(C₆-C₂₆)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₆)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₆)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₆)-Aryl, -(C₆-C₂₆)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte - (C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl, -O-(C₆-C₂₆)-Aryl, -S-Alkyl, -S-Aryl.

In einer Ausführungsform stehen R⁹', R¹⁰', R¹¹', R¹²', R⁹", R¹⁰", R¹¹", R¹²" für -(C₆-C₂₀)-Aryl.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl.

In einer Ausführungsform stehen R⁹', R¹⁰', R¹¹', R¹²', R⁹", R¹⁰", R¹¹", R¹²" für Phenyl.

In einer Ausführungsform stehen R¹ und R⁸ nicht für den gleichen Rest.

In einer Ausführungsform stehen R² und R⁷ nicht für den gleichen Rest.

In einer Ausführungsform stehen R³ und R⁶ nicht für den gleichen Rest.

In einer Ausführungsform stehen R⁴ und R⁵ nicht für den gleichen Rest.

In einer Ausführungsform stehen R⁹', R¹⁰', R¹¹', R¹²', R⁹", R¹⁰", R¹¹", R¹²" für den gleichen Rest.

In einer Ausführungsform stehen R⁹', R¹⁰', R¹¹', R¹²' für den gleichen Rest.

In einer Ausführungsform stehen R⁹", R¹⁰", R¹¹", R¹²" für den gleichen Rest.

In einer Ausführungsform weist die Verbindung eine der folgenden Formeln auf:

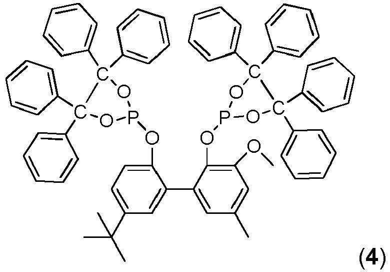

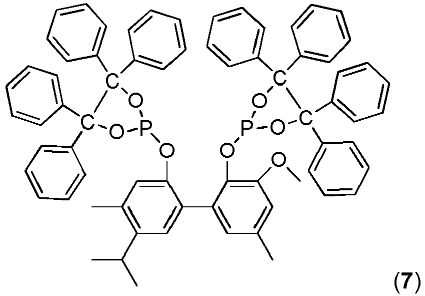

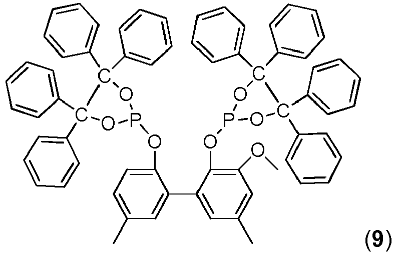

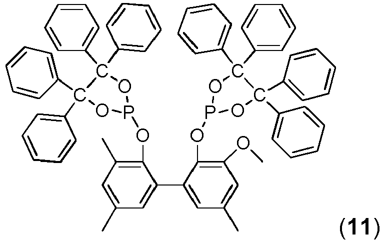

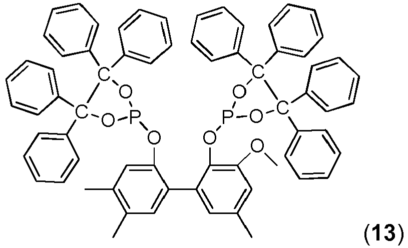

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

Es kann hierbei auch ein Überschuss an Liganden verwendet werden und nicht zwangläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2-oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen und einer Figur näher erläutert.

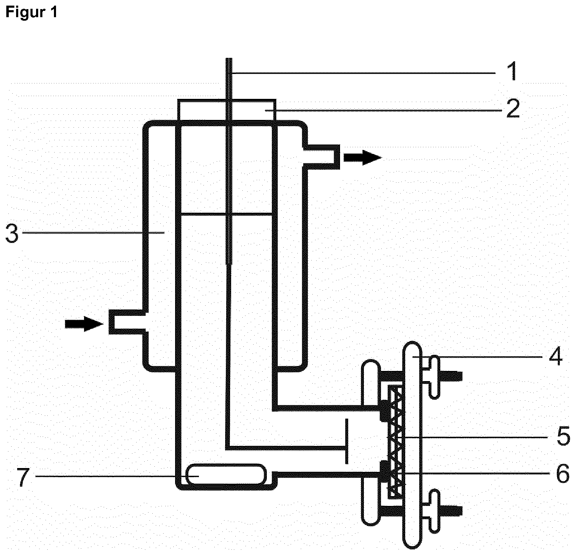

Die Figur 1 zeigt eine Reaktionsapparatur, in welcher die Kupplungsreaktion zu den entsprechenden unsymmetrischen Biarylen durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.
Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Schmelzpunkte

Schmelzpunkte wurden mit Hilfe des Schmelzpunktbestimmungsgerätes SG 2000 der Firma HW5, Mainz gemessen und sind unkorrigiert.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. El-Massenspektren sowie die hochaufgelösten El-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

### Synthese der Chlorophosphite

6-Chlorodibenzo[*d*,*f*[1,3,2]dioxaphosphepin wurde nach DE 10 2008 043 584 und 2-Chloro-4,4,5,5-tetraphenyl-1,3,2-dioxaphospholan nach DE 10 2006 058 682 hergestellt.

### Synthese der unsymmetrischen Biaryle

Die unsymmetrischen Biaryle wurden durch ein elektrochemisches Verfahren durch Kupplung von zwei Phenolen bzw. von einem Naphthol mit einem Phenol, welche sich in ihrem Oxidationspotential unterscheiden, hergestellt. Siehe hierzu auch B. Elsler, D. Schollmeyer, K. M. Dyballa, R. Franke, S. R. Waldvogel, "Metall- und reagensfreie hochselektive anodische Kreuzkupplung von Phenolen", Angew. Chem., 2014, DOI: 10.1002/ange.201400627

### Allgemeine Arbeitsvorschrift:

Die Kupplungsreaktion wurde in einer Apparatur durchgeführt, wie sie in Figur 1 dargestellt ist. 5 mmol des ersten Phenols mit einem Oxidationspotential *E_{Ox}1* werden mit 15 mmol des zweiten Phenols mit einem Oxidationspotential *E_{Ox}2* in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, 10⁻³ mbar).

### Elektrodenmaterial

| | |
|---|---|
| Anode: | Bor-dotierter Diamant (BDD) auf Si |
| Kathode: | Ni-Netz |

### Elektrolysebedingungen:

| | |
|---|---|
| Temperatur [T]: | 50 °C |
| Stromstärke [I]: | 15 mA |
| Stromdichte [j]: | 2.8 mA/cm² |
| Ladungsmenge [Q]: | 2 F/mol Unterschusskomponente |
| Klemmspannung [Uₘₐₓ]: | 3-5 V |

Die Synthese der Biaryle erfolgte gemäß der oben beschriebenen allgemeinen Arbeitsvorschrift, und in einer Reaktionsapparatur, wie sie in Figur 1 dargestellt ist.

### 2,2'-Dihydroxy-4',5-dimethyl-5'-(methylethyl)-3-methoxybiphenyl

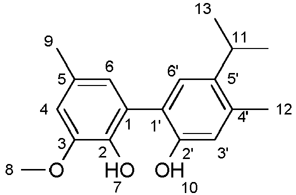

Es wurden 0.69 g (5 mmol, 1.0 Äquiv.) 4-Methylguajacol und 2.28 g (15 mmol, 3.0 Äquiv.) 3-Methyl-4-(methylethyl)phenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten. Ausbeute: 716 mg (50%, 2.5 mmol)
GC (Methode *hart,* HP-5): t_{R}= 14.87 min
R_{f}(CH:EE= 4:1)= 0.43
mₚ= 126.8°C (aus CH umkristallisiert)
¹H-NMR (600 MHz, DMSO) δ= 1.17-1.12 (m, 6H, 13-H), 2.24 (m, 6H, 9-H/12-H), 3.01 (dt, 1H, 11-H), 3.79 (s, 3H, 8-H), 6.55 (s, 1H, 6-H), 6.66 (d, 1H, 6'-H), 6.73 (d, 1H, 4-H), 6.96 (s, 1H, 3'-H), 8.16 (s, 1H, 7-H), 8.84 (s, 1H, 10-H);
Kopplungen: ⁴J_{4-H, 6-H}= 2.2 Hz, ⁴J_{6'-H, 11-H}= 2.9 Hz, ³J_{11-H, 13-H}= 6.8 Hz;
¹³C-NMR (151 MHz, DMSO) δ= 18.73, 20.80 (C-9/C-12), 23.54 (C-13), 28.10 (C-11), 55.78 (C-8), 111.23 (C-4), 117.34 (C-6'), 123.42 (C-1'), 123.49 (C-6), 126.43 (C-1), 127.36 (C-5), 127.49 (C-3'), 134.40 (C-5'), 136.62 (C-4'), 141.12 (C-2), 147.65 (C-3), 151.69 (C-2').
HRMS für C₁₈H₂₂O₃ (ESI+) [M+Na⁺]: ber: 309.1467, gef.: 309.1457
MS (EI, GCMS): m/z(%): 286 (50) [M]⁺˙, 271 (100) [M-CH₃˙]⁺, 244 (22) [M-C₃H₆˙]⁺. Elementaranalyse für C₁₈H₂₂O₃: ber: C: 75.50%, H: 7.74%, gef.: C: 75.01%, H: 7.70%.

### 2,2'-Dihydroxy-5,5'-dimethyl-3-methoxybiphenyl

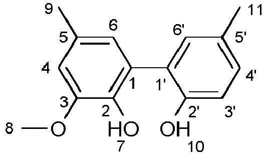

Es wurden 1.66 g (12 mmol, 1.0 Äquiv.) 4-Methylguajacol und 3.91 g (36 mmol, 3.0 Äquiv.) 4-Methylphenol in 65 mL HFIP und 14 mL MeOH gelöst, 1.63 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 440 mg (36%, 1.8 mmol)
GC (Methode *hart,* HP-5): t_{R}= 13.56 min
R_{f}(CH:EE= 4:1)= 0.38
mₚ= 162.0 °C (aus CH umkristallisiert)
¹H-NMR (400 MHz, DMSO) δ= 2.18 (s, 3H, 9-H/11-H), 2.21 (s, 3H, 9-H/11-H), 3.76 (s, 3H, 8-H), 6.53 (s, 1H, 6-H), 6.71 (s, 1H, 4-H), 6.75 (d, 1H, 3'-H), 6.86-6.94 (m, 2H, 4'-H/6'-H), 8.53 (bs, 1H, 7-H/12-H);
Kopplungen: ³J_{3'-H, 4'-H}= 8.4 Hz;
¹³C-NMR (101 MHz, DMSO) δ= 20.21, 20.77 (C-9/C-11), 55.79 (C-8), 111.36 (C-4), 115.69 (C-3'), 123.50 (C-6), 125.72 (C-1'), 126.16 (C-1), 127.20 (C-5), 127.30 (C-5'), 128.50 (C-6'), 131.83 (C-4'), 141.20 (C-2), 147.61 (C-3), 152.11 (C-2').
HRMS für C₁₅H₁₆O₃ (ESI+) [M+Na⁺]: ber: 267.0997, gef.: 267.0999
MS (EI, GCMS): m/z(%): 244 (100) [M]⁺˙, 229 (64) [M-CH₃˙]⁺.
Elementaranalyse für C₁₅H₁₆O₃: ber: C: 73.75%, H: 6.60%, gef.: C: 73.81%, H: 6.54%.

### 2,2'-Dihydroxy-3-methoxy-3',5,5'-trimethyl-biphenyl

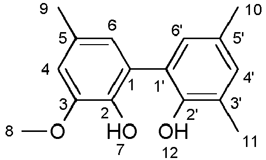

Es wurden 0.70 g (6 mmol, 1.0 Äquiv.) 4-Methylguajacol und 2.08 g (17 mmol, 3.0 Äquiv.) 2,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als leichtgelber Feststoff erhalten.
Ausbeute: 663 mg (45%, 2.5 mmol)
GC (Methode *hart,* HP-5): t_{R}= 13.97 min
R_{f}(CH:EE= 4:1)= 0.29
mₚ= 119.7 °C (aus DCM/CH umkristallisiert)
¹H-NMR (400 MHz, CDCl₃) δ= 2.34 (s, 3H, 10-H), 2.35 (s, 3H, 11-H), 2.38 (s, 3H, 9-H), 3.94 (s, 3H, 8-H), 6.16 (s, 1H, 12-H), 6.20 (s, 1H, 7-H), 6.76 (d, 1H, 4-H), 6.78 (d, 1H, 6-H), 6.98 (d, 1H, 6'-H), 7.03 (d, 1H, 4'-H);
Kopplungen: ⁴J_{4-H, 6-H}= 1.7 Hz, ⁴J_{4'-H, 6-H}= 2.1 Hz;
¹³C-NMR(101 MHz, CDCl₃) δ= 16.51 (C-9), 20.54 (C-10), 21.20 (C-11), 56.12 (C-8), 110.92 (C-4), 123.95 (C-6), 124.13 (C-1), 124.64 (C-1'), 126.18 (C-3'), 128.82 (C-6'), 129.59 (C-5'), 130.40 (C-5), 131.40 (C-4'), 139.46 (C-2), 146.35 (C-3), 149.42 (C-2').
HRMS für C₁₈H₁₆O₃ (ESI+) [M+Na⁺]: ber: 281.1154, gef.: 281.1152
MS (El, GCMS): m/z(%): 242 (100) [M]⁺˙, 227 (38) [M-CH₃˙]⁺.
Elementaranalyse für C₁₆H₁₈O₃: ber: C: 68.31%, H: 6.45%, gef.: C: 68.29%, H: 6.40%.

### 2,2'-Dihydroxy-3-methoxy-5-methyl-4'-(dimethylethyl)biphenyl

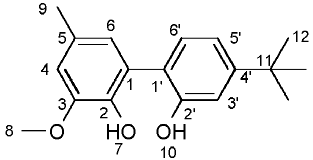

Es wurden 0.69 g (5 mmol, 1.0 Äquiv.) 4-Methylguajacol und 2.25 g (15 mmol, 3.0 Äquiv.) 3-tert-Butylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten. Ausbeute: 808 mg (63%, 3.1 mmol)
GC (Methode *hart,* HP-5): t_{R}= 13.97 min
R_{f}(CH:EE= 4:1)= 0.29
mₚ= 160.3 °C (aus DCM/CH umkristallisiert)
¹H-NMR (400 MHz, CDCl₃) δ= 1.37 (s, 9H, 12-H), 2.36 (s, 3H, 9-H), 3.94 (s, 3H, 8-H), 6.25 (s, 1H, 7-H), 6.48 (s, 1H, 10-H), 6.75 (d, 1H, 6-H), 6.79 (d, 1H, 4-H), 7.08 (dd, 1H, 5'-H), 7.12 (d, 1H, 3'-H), 7.27 (d, 1H, 6'-H);
Kopplungen: ⁴J_{4-H, 6-H}= 1.7 Hz; ³J_{5'-H, 6'-H}= 8.0 Hz, ⁴J_{3-H, 5-H}= 1.7 Hz;
¹³C-NMR(101 MHz, CDCl₃) δ= 21.24 (C-9), 31.31 (C-12), 34.58 (C-11), 56.15 (C-8), 110.79 (C-4), 114.94 (C-3'), 118.30 (C-5'), 122.37 (C-1'), 123.88 (C-1), 123.94 (C-6), 130.45 (C-6'), 130.53 (C-4'), 139.24 (C-5), 146.32 (C-3), 152.91 (C-2'), 153.13 (C-2).
HRMS für C₁₅H₁₆O₄ (ESI+) [M+Na⁺]: ber: 309.1467, gef.: 309.1466
MS (El, GCMS): m/z(%): 242 (100) [M]⁺˙, 227 (38) [M-CH₃˙]⁺.
Elementaranalyse für C₁₈H₂₂O₃: ber: 75.50%, H: 7.74%, gef.: C: 75.41%, H: 7.72%.

### 2,2'-Dihydroxy-4',5-dimethy-3-methoxylbiphenyl

Es wurden 0.70 g (5 mmol, 1.0 Äquiv.) 4-Methylguajacol und 1.65 g (15 mmol, 3.0 Äquiv.) 3-Methylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und zwei Kreuzkupplungsprodukte als farblose Feststoffe erhalten. 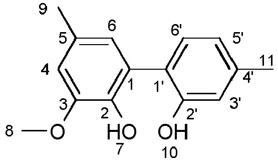 Ausbeute: 266 mg (21%, 1.1 mmol)
GC (Methode *hart,* HP-5): t_{R}= 13.72 min
R_{f}(CH:EE= 4:1)= 0.25
mₚ= 136.2 °C (aus DCM/CH umkristallisiert)
¹H-NMR (400 MHz, CDCl₃) δ= 2.35 (s, 3H, 9-H/11-H), 2.37 (s, 3H, 9-H/11-H), 3.94 (s, 3H, 8-H), 6.17 (s, 1H, 10-H), 6.35 (s, 1H, 2-H), 6.74 (d, 1H, 4-H), 6.76 (s, 1H, 6-H), 6.88-6.83 (m, 1H, 5'-H), 6.90 (d, 1H, 3'-H), 7.21 (d, 1H, 6'-H);
Kopplungen: ⁴J_{4-H, 6-H}= 1.8 Hz, ³J_{5'-H, 6'-H}= 7.7 Hz, ⁴J_{3'-H, 5'-H}= 1.5 Hz;
¹³C-NMR (101 MHz, CDCl₃) δ= 21.11, 21.20 (C-9/C-11), 56.13 (C-8), 110.81 (C-4), 118.25 (C-3'), 121.97 (C-5'), 122.39 (C-1), 123.77 (C-1'), 123.85 (C-6), 130.50 (C-5), 130.68 (C-6'), 139.30 (C-4'), 139.54 (C-2), 146.31 (C-3), 153.33 (C-2').
HRMS für C₁₅H₁₆O₃ (ESI+) [M+Na⁺]: ber: 267.0997, gef.: 267.1006
MS (El, GCMS): m/z(%): 244 (100) [M]⁺˙, 229 (18) [M-CH₃˙]⁺'.
Elementaranalyse für C₁₅H₁₆O₃: ber: C: 73.75%, H: 6.60%, gef.: C: 73.70%, H: 6.68%.

### 2,2'-Dihydroxy-3-methoxy-4'-5,5'-trimethylbiphenyl

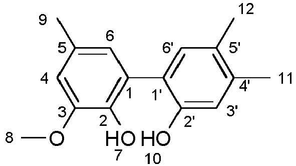

Es wurden 0.69 g (5 mmol, 1.0 Äquiv.) 4-Methylguajacol und 1.83 g (15 mmol, 3.0 Äquiv.) 3,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 688 mg (52%, 2.6 mmol)
GC (Methode *hart,* HP-5): t_{R}= 14.52 min
R_{f}(CH:EE= 4:1)= 0.29
mₚ= 152.3 °C (aus DCM/CH umkristallisiert)
¹H-NMR (400 MHz, CDCl₃) δ= 12.25 (s, 3H, 11-H), 2.28 (s, 3H, 12-H), 2.36 (s, 3H, 9-H), 3.93 (s, 3H, 8-H), 6.19 (s, 1H, 7-H), 6.25 (s, 1H, 10-H), 6.73 (d, 1H, 4-H), 6.76 (s, 1H, 6-H), 6.88 (s, 1H, 3'-H), 7.08 (s, 1H, 6'-H);
Kopplungen: ⁴J_{4-H, 6-H}= 1.7 Hz;
¹³C-NMR (101 MHz, CDCl₃) δ= 18.89 (C-11), 19.60 (C-12), 21.24 (C-9), 56.14 (C-8), 110.74 (C-4), 118.93 (C-3'), 122.54 (C-1), 123.82 (C-6), 123.97 (C-1'), 129.03 (C-5), 130.46 (C-4'), 131.69 (C-6'), 137.94 (C-5'), 139.26 (C-2), 146.31 (C-3), 151.36 (C-2').
HRMS für C₁₆H₁₈O₃ (ESI+) [M+Na⁺]: ber: 281.1154, gef.: 281.1157
MS (El, GCMS): m/z(%): 258 (100) [M]⁺˙, 243 (10) [M-CH₃˙]⁺.
Elementaranalyse für C₁₆H₁₈O₃: ber: 74.39%, H: 7.02%, gef.: C: 74.32%, H: 7.20%

### 2,2'-Dihydroxy-5'-isopropyl-3-methoxy-5-methylbiphenyl

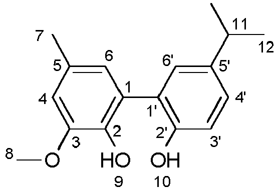

Es wurden 0.69 g (5 mmol, 1.0 Äquiv.) 4-Methylguajacol und 2.05 g (15 mmol, 3.0 Äquiv.) 4-Isopropylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 0.53 g (39%, 1.9 mmol)
GC (Methode *hart,* HP-5): t_{R}= 14.23 min
R_{f}(CH:EE= 4:1)= 0.30
¹H-NMR (400 MHz, CDCl₃) δ= 1.27 (m, 6H), 2.36 (s, 3H), 2.91 (dt, J= 13.8, 6.9, 6.9 Hz, 1H), 3.94 (s, 3H), 6.13-6.27 (m, 2H), 6.82-6.65 (m, 1H), 6.25 (m, 2H), 6.75 (s, 1H), 6.77 (s, 1H), 6.99 (d, J= 8.1 Hz, 1H), 7.19-7.12 (m, 2H);
¹³C-NMR (101 MHz, CDCl₃) δ= 21.25, 24.27, 33.40, 56.18, 110.92, 117.60, 123.91, 124.23, 125.07, 127.29, 128.80, 130.57, 139.29, 141.42, 146.31, 151.51.
HRMS für C₁₇H₂₀O₃ (ESI+) [M+Na⁺]: ber: 295.1310, gef.: 295.1297
MS (El, GCMS): m/z(%): 272 (80) [M]⁺˙, 257 (100) [M-CH₃˙]⁺.

### 2,2'-Dihydroxy-3-methoxy-5-methyl-5'-tert-butylbiphenyl

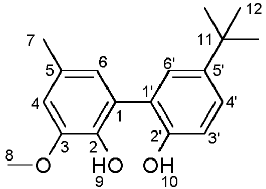

Es wurden 0.69 g (5 mmol, 1.0 Äquiv.) 4-Methylguajacol und 2.26 g (15 mmol, 3.0 Äquiv.) 4-*Tert-*butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.48 g (34%, 1.7 mmol)
GC (Methode *hart,* HP-5): t_{R}= 14.52 min
R_{f}(CH:EE= 4:1)= 0.24
¹H-NMR (400 MHz, CDCl₃) δ= 1.34 (s, 9H), 2.37 (s, 3H), 3.94 (s, 3H), 6.17 (s, 1H), 6.24 (s, 1H), 6.75 (s, 1H), 6.77 (s, 1H), 6.99 (d, J= 8.4 Hz, 1H), 7.31-7.29 (m, 1H), 7.33 (dd, J= 8.4, 2.5 Hz, 1H).
¹³C-NMR (101 MHz, CDCl₃) δ= 21.28, 31.61, 34.20, 56.18, 110.91, 117.25, 123.92, 124.41, 124.63, 126.38, 127.78, 130.58, 139.32, 143.70, 146.32, 151.22.
HRMS für C₁₈H₂₂O₃ (ESI+) [M+Na⁺]: ber: 309.1467, gef.: 309.1476
MS (El, GCMS): m/z(%): 286 (28) [M]⁺˙, 271 (100) [M-CH₃˙]⁺.

### 2,2'-Dihydroxy-3',5'-di-tert-butyl-5-methyl-3-methoxybiphenyl

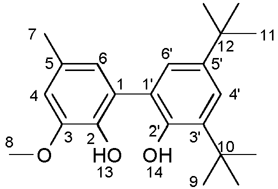

Es wurden 0.69 g (5 mmol, 1.0 Äquiv.) 4-Methylguajacol und 3.12 g (15 mmol, 3.0 Äquiv.) 2,4-Di*tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 0.41 g (24%, 1.2 mmol)
GC (Methode*hart*, HP-5): t_{R}= 15.15 min
R_{f}(CH:EE= 9:1)= 0.35
mₚ= 120.2 °C (inn-Pentan umkristallisiert)
¹H-NMR (400 MHz, CDCl₃) δ= 1.36 (s, 9H), 1.50 (s, 9H), 2.38 (s, 3H), 3.96 (s, 3H), 6.00 (s, 1H), 6.05 (s, 1H), 6.77 (s, 1H), 7.16 (d, J= 2.5 Hz, 1H), 7.39 (d, J= 2.5 Hz, 1H).
¹³C-NMR (101 MHz, CDCl₃) δ= 21.23, 29.88, 31.69, 34.40, 35.23, 56.17, 111.03, 123.96, 124.17, 125.09, 125.50, 130.42, 136.73, 139.72, 142.36, 146.45, 149.82.
MS (El, GCMS): m/z(%): 342 (22) [M]⁺˙, 327 (100) [M-CH₃˙]⁺.

### 2,2'-Dihydroxy-3',5-dimethyl-3-methoxy-5'-tert-butylbiphenyl

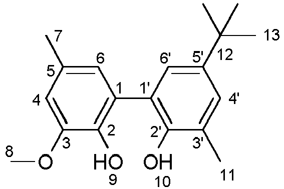

Es wurden 0.69 g (5 mmol, 1.0 Äquiv.) 4-Methylguajacol und 2.47 g (15 mol, 3.0 Äquiv.) 2-Methyl-4-*tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.69 g (46%, 2.3 mmol)
GC (Methode *hart,* HP-5): t_{R}= 14.79 min
R_{f}(CH:EE= 4:1)= 0.33
¹H-NMR (400 MHz, CDCl₃) δ= 1.37 (s, 9H), 2.39 (d, J= 2.4 Hz, 6H), 3.94 (s, 3H), 6.15 (s, 1H), 6.17 (s, 1H), 6.77 (s, 1H), 6.79 (s, 1H), 7.17 (d, J= 2.5 Hz, 1H), 7.24 (d, J= 2.4 Hz, 1H);
¹³C-NMR (101 MHz, CDCl₃) δ= 16.90, 21.28, 31.67, 34.12, 56.16, 110.94, 124.02, 124.17, 124.59, 125.41, 125.65, 127.86, 130.47, 139.50, 143.07, 146.40, 149.41.
MS (El, GCMS): m/z(%): 300 (18) [M]⁺˙, 285 (100) [M-CH₃˙]⁺.

### 2,2'-Dihydroxy-3-methoxy-5-methyl-5'-(1-methylethyl)biphenyl

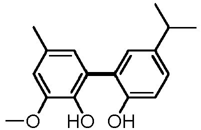

Es wurden 0.69 g (5 mmol, 1.0 eq.) 4-Methylguajacol und 2.05 g (15 mmol, 3.0 eq.) 4-isopropylphenol und 0.68 g Methyltriethylammoniummethylsulfat (MTES) in 27 mL HFIP + 6 mL MeOH Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 39%, 527 mg, 1.9 mmol.
R_{f} (Cyclohexane:Ethylacetat = 4:1)= 0.30; ¹H NMR (400 MHz, CDCl₃) δ= 1.27 (m, 6H), 2.36 (s, 3H), 2.91 (sept, *J*= 6.9 Hz, 1H), 3.94 (s, 3H), 6.13-6.27 (m, 2H), 6.65-6.82 (m, 2H), 6.99 (d, *J=* 8.1 Hz, 1H), 7.12-7.19 (m, 2H); ¹³C NMR (101 MHz, CDCl₃) δ= 21.37, 24.39, 33.53, 56.31, 111.04, 117.73, 124.04, 124.36, 125.20, 127.42, 128.93, 130.70, 139.42, 141.55, 146.44, 151.64. HRMS für C₁₇H₂₀O₃ (ESI+) [M+Na⁺]: calc.: 295.1310, found: 295.1297; MS (El, GCMS): m/z(%): 272 (80) [M]⁺˙, 257 (100) [M-CH₃˙]⁺.

### 2,2'-Dihydroxy-3-methoxy-5-methyl-4'-(methylethyl)biphenyl

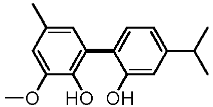

Es wurden 0.69 g (5 mmol, 1.0 eq.) 4-Methylguajacol und 2.065 g (15 mmol, 3.0 eq.) 3-isopropylphenol und 0.68 g Methyltriethylammoniummethylsulfat (MTES) in 33 mL HFIP gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten (Ausbeute: 52%, 705 mg, 2.6 mmol). R_{f} (Cyclohexane:Ethylacetat = 4:1)= 0.29; ¹H NMR (400 MHz, CDCl₃) δ= 1H NMR (400 MHz, CDCl3) δ 1.27 (s, 3H), 1.29 (s, 3H), 2.34 (s, 3H), 2.91 (sept, *J*= 7.0 Hz, 1H), 3.94 (s, 3H), 6.15 (s, 1H), 6.35 (s, 1H), 6.73 (d, *J*= 1.8 Hz, 1H), 6.75-6.77 (m, 1H), 6.90 (dd, *J*= 7.9 Hz, 1.8 Hz, 1H), 6.94 (d, *J*= 1.7 Hz, 1H), 7.23 (d, *J*= 7.8 Hz, 1H). ¹³C NMR (101 MHz, CDCl₃) δ= ¹³C NMR (101 MHz, CDCl₃) δ 21.36, 24.02, 33.92, 56.30, 77.16, 110.91, 115.77, 119.56, 122.81, 124.00, 124.08, 130.65, 130.84, 139.38, 146.43, 150.72, 153.54. HRMS für C₁₇H₂₀O₃ (ESI+) [M+Na⁺]: calc.: 295.1310, found: 295.1305; MS (El, GCMS): m/z(%): 272 (100) [M]⁺˙, 257 (50) [M-CH₃˙]⁺. Elementaranalyse für C₁₇H₂₀O₃: calc.: 74.97%, H: 7.40%, found: C: 75.05%, H: 7.36%.

### 2,2'-Dihydroxy-4',5-dimethyl-3-methoxybiphenyl

Es wurden 0.28 g (2 mmol, 1.0 eq.) 4-Methylguaiacol, 1.22 g (6 mmol, 3.0 eq.) 3-Methylphenyl und 0.77 g MTBS 25 mL HFIP gelöst und der Elektrolyt in die Beaker-typ Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und führte zu den beiden Kreuzkupplungsprodukten als farbloses und viskoses Öl.

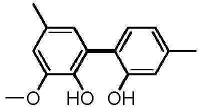

Ausbeute: 21%, 266 mg, 1.1 mmol; R_{f} (Cyclohexane:Ethylacetat = 4:1)= 0.25; mₚ= 136.2 °C (kristallisiert aus Dichloromethan/Cyclohexan); ¹H NMR (400 MHz, CDCl₃) δ= 2.35 (s, 3H), 2.37 (s, 3H,), 3.94 (s, 3H), 6.17 (s, 1H), 6.35 (s, 1H), 6.74 (d, *J=* 1.8 Hz, 1H), 6.76 (s, 1H), 6.88-6.83 (m, 1H), 6.90 (d, 1H, *J=* 1.5 Hz), 7.21 (d, 1H, *J= 7.7* Hz); ¹³C NMR (101 MHz, CDCl₃) δ= 21.11, 21.20 56.13, 110.81, 118.25, 121.97, 122.39, 123.77, 123.85, 130.50, 130.68, 139.30, 139.54, 146.31, 153.33. HRMS für C₁₅H₁₆O₃ (ESI+) [M+Na⁺]: calc.: 267.0997, found: 267.1006; MS (El, GCMS): m/z(%): 244 (100) [M]⁺˙, 229 (18) [M-CH₃˙]^{+,}. Elementaranalyse für C₁₅H₁₆O₃: calc.: C: 73.75%, H: 6.60%, found: C: 73.70%, H: 6.68%.

### 2,2'-Dihydroxy-5,5'-dimethyl-3'-(1,1-dimethylethl)-3-methoxybiphenyl

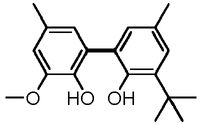

Es wurden 0.69 g (5 mmol, 1.0 eq.) 4-Methylguaiacol, 2.47 g (15 mmol, 3.0 eq.) 4-Methyl-2-*tert-*butylphenol und 0.68 g Methyltriethylammoniummethylsulfat (MTES) in 27 mL HFIP + 6 mL MeOH gelöst gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbes Öl erhalten (Ausbeute: 36%, 545 mg, 1.8 mmol).
R_{f} (Cyclohexane:Ethylacetat = 9:1)= 0.36; ¹H NMR (400 MHz, CDCl₃) δ= 1.46 (s, 9H), 2.34 (m, 6H), 3.93 (s, 3H), 5.99 (s, 1H), 6.01 (s, 1H), 6.74 (s, 2H), 6.96 (d, *J=* 1.9 Hz, 1H), 7.14 (d, *J=* 1.9 Hz, 1H); ¹³C NMR (101 MHz, CDCl₃) δ= 21.05, 21.32, 29.96, 35.05, 56.30, 77.16, 111.21, 124.18, 124.24, 125.92, 127.67, 129.15, 129.22, 130.51, 137.57, 139.87, 146.57, 150.10. HRMS für C₂₂H₃₆O₃ (ESI+) [M+Na⁺]: calc.: 323.1623, found: 323.1618; MS (El, GCMS): m/z(%): 300 (100) [M]⁺˙, 285 (100) [M-CH₃˙]⁺.

### Synthese der Liganden

### 2,2'-((5'-(tert-Butyl)-3-methoxy-5-methyl-[1,1'-biphenyl]-2,2'-diyl)bis(oxy))bis(4,4,5,5-tetraphenyl-1,3,2-dioxaphospholan).

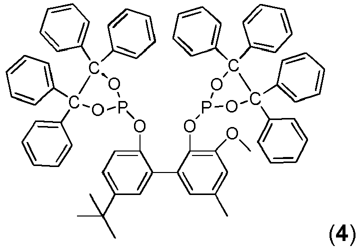

Eine Lösung von 4'-(*tert*-Butyl)-3-methoxy-5-methyl-[1,1'-biphenyl]-2,2'-diol (0,327 g; 1,143 mmol) in THF (5 ml) wurde mit 2 Äquivalenten n-Butyllithium in Hexan (4,3 ml) bei -20 °C versetzt. Die Mischung wurde für 20 min bei -20 °C gerührt, und nach Erwärmen auf Raumtemperatur eine Lösung von 2-Chloro-4,4,5,5-tetraphenyl-1,3,2-dioxaphospholan (0,985 g; 2,287 mmol) in THF (8 ml) zugegeben. Die Reaktionsmischung wurde über Nacht gerührt und das Lösungsmittel im Vakuum abgezogen. Toluol (20 ml) wurde zugegeben und die resultierende Lösung filtriert. Das trübe Filtrat wurde nochmals über Kieselgel filtriert und das Lösungsmittel im Vakuum entfernt. Der erhaltene Feststoff wurde bei 50 °C / 0,1 mbar getrocknet und dann aus heißem Acetonitril (11 ml) umkristallisiert. Ausbeute: 0,840 g (0,781 mmol; 68 %).
Elementaranalyse (ber. für C₇₆H₆₆O₇P₂ = 1075,19 g/ mol) C 78,36 (78,20); H 5,75 (5,62); P 5,95 (5,76) %.
³¹P-NMR (CD₂Cl₂): 139,3 (d, *J*_{PP}= 13,6 Hz); 145,2 (d, *J*_{PP}= 13,6 Hz) ppm.
¹H-NMR (CD₂Cl₂): 1,26 (m, 9 H); 2,38 (m, 3 H); 3,95 (s, 3 H); 6,54 (m, 1 H, Hₐᵣₒₘ); 6,70 (m, 1 H, Hₐᵣₒₘ); 6,91 (m, 1 H, Hₐᵣₒₘ); 6,98-7,04 (m, 4 H, Hₐᵣₒₘ); 7,05-7,13 (m, 20 H, Hₐᵣₒₘ); 7,13-7,21 (m, 10 H, Hₐᵣₒₘ); 7,33-7,40 (m, 4 H, Hₐᵣₒₘ); 7,49-7,55 (m, 4 H, Harom) ppm.
¹³C-NMR (CD₂Cl₂): 31,5; 57,4; 94,9 (d, *J*_{CP}= 9,0 Hz); 95,3 (d, *J*_{CP}= 8,5 Hz); 114,2; 118,9 (d, *J*_{CP}= 9,0 Hz); 121,1; 125,6; 127,2; 127,3; 127,4; 127,5; 127,6; 127,9; 127,9; 129,1 (d, *J*_{CP}= 3,0 Hz); 129,2 (d, *J*_{CP}= 3,0 Hz); 130,0; 130,3; 131,3; 132,0; 133,4; 138,5 (d, *J*_{CP}= 9,0 Hz); 142,6 (d, *J*_{CP}= 4,0 Hz); 142,8; 143,0; 143,0; 148,6 (d, *J*_{CP}= 6,8 Hz); 151,0 (d, *J*_{CP}= 3,4 Hz); 152,6 ppm.

### 2,2'-((5'-Isopropyl-3-methoxy-4',5-dimethyl-[1,1'-biphenyl]-2,2'-diyl)bis(oxy))bis(4,4,5,5-tetraphenyl-1,3,2-dioxaphospholan).

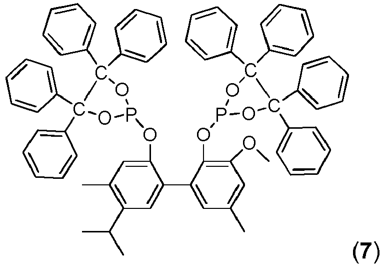

Eine Lösung von 5'-Isopropyl-3-methoxy-4',5-dimethyl-[1,1'-biphenyl]-2,2'-diol (0,330 g; 1,153 mmol) in THF (5 ml) wurde mit 2 Äquivalenten n-Butyllithium, gelöst in Hexan (4,3 ml), bei - 20°C versetzt. Die Mischung wurde für 20 min bei -20 °C gerührt, und nach Erwärmen auf Raumtemperatur eine Lösung von 2-Chloro-4,4,5,5-tetraphenyl-1,3,2-dioxaphospholan (0,994 g; 2,307 mmol) in THF (8 ml) zugegeben. Die Reaktionsmischung wurde über Nacht gerührt und das Lösungsmittel im Vakuum entfernt. Toluol (20 ml) wurde zugegeben und die resultierende Lösung über Kieselgel filtriert. Das Filtrat wurde im Vakuum bis zur Trockne eingeengt. Der erhaltene Feststoff wurde bei 50 °C / 0,1 mbar getrocknet und dann aus heißem Acetonitril (12 ml) umkristallisiert. Ausbeute: 0,782 g (0,728 mmol; 63 %). Elementaranalyse (ber. für C₇₀H₆₀O₇P₂ = 1075,19 g/ mol) C 78,18 (78,20); H 5,69 (5,62); P 5,87 (5,76) %.
³¹P-NMR (CD₂Cl₂): 139,3 (d, *J*_{PP}= 9,1 Hz); 145,0 (d, *J*_{PP}= 9,1 Hz) ppm.
¹H-NMR (CD₂Cl₂): 1,21 (m, 6 H); 2,27 (m, 3 H); 2,42 (m, 3 H); 3,96 (s, 3 H); 6,07 (m, 1 H, Hₐᵣₒₘ); 6,68 (m, 1 H, Harom); 6,93 (m, 3 H, Hₐᵣₒₘ); 6,95-7,05 (m, 7 H, Hₐᵣₒₘ); 7,05-7,16 (m, 18 H, Hₐᵣₒₘ); 7,16-7,23 (m, 7 H, Hₐᵣₒₘ); 7,30-7,38 (m, 4 H, Hₐᵣₒₘ); 7,49-7,56 (m, 4 H, Hₐᵣₒₘ) ppm.
¹³C-NMR (CD₂Cl₂): 19,1; 21,5; 23,5; 29,3; 57,5; 94,8 (d, *J*_{CP}= 8,8 Hz); 95,5 (d, *J*_{CP}= 8,2 Hz); 114,2; 123,3 (d, *J*_{CP}= 10,0 Hz); 125,4; 127,1; 127,2; 127,3; 127,3; 127,4; 127,6; 128,3; 128,7 (d, *J*_{CP}= 3,4 Hz); 129,0; 129,3; 129,9; 130,4 (m); 132,2 (d, *J*_{CP}= 3,0 Hz); 133,5; 136,1; 138,5 (d, *J*_{CP}= 6,8 Hz); 142,5; 142,7; 142,8; 143,0; 143,1 (d, *J*_{CP}= 4,6 Hz); 146,1 (d, *J*_{CP}= 5,2 Hz); 151,0 (d, *J*_{CP}= 3,6 Hz) ppm.

### 2,2'-((3-Methoxy-5,5'-dimethyl-[1,1'-biphenyl]-2,2'-diyl)bis(oxy))bis(4,4,5,5-tetraphenyl-1,3,2 dioxaphospholan).

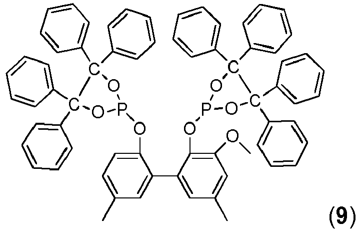

Eine Lösung von 3-Methoxy-5,5'-dimethyl-[1,1'-biphenyl]-2,2'-diol (0,241 g; 0,986 mmol) in THF (4 ml) wurde mit 2 Äquivalenten n-Butyllithium, gelöst in Hexan (3,7 ml), bei -20 °C versetzt. Die Mischung wurde zunächst für 20 min bei -20 °C gerührt und nach Erwärmen auf Raumtemperatur eine Lösung von 2-Chloro-4,4,5,5-tetraphenyl-1,3,2-dioxaphospholan (0,850 g; 1,973 mmol) in THF (7 ml) zugegeben. Die Reaktionsmischung wurde über Nacht gerührt und das Lösungsmittel im Vakuum eingeengt. Der Rückstand wurde mit Toluol (20 ml) verrührt und die resultierende Suspension über Kieselgel filtriert. Das Filtrat wurde im Vakkum zur Trockne eingeengt. Der erhaltene Feststoff wurde bei 50 °C / 0,1 mbar getrocknet und dann aus heißem Acetonitril (11 ml) umkristallisiert. Die zurückgebliebene Substanz wurde durch Säulenchromatografie gereinigt (Hexan/Toluol, 1:2, R*_{f}*= 0,3). Ausbeute: 0,430 g (0,416 mmol; 43 %).
Elementaranalyse (ber. für C₆₇H₅₄O₇P₂ = 1033,10 g/ mol) C 77,64 (77,89); H 5,50 (5,27); P 6,05 (6,00) %.
³¹P-NMR (CD₂Cl₂): 139,9 (d, *J*_{PP}= 17,5 Hz); 145,1 (d, *J*_{PP}= 17,5 Hz) ppm.
¹H-NMR (CD₂Cl₂): 26 (m, 3 H); 2,35 (m, 3 H); 3,91 (s, 3 H); 6,57 (m, 1 H, Hₐᵣₒₘ); 6,63 (m, 1 H, Hₐᵣₒₘ); 6,88 (m, 1 H, Hₐᵣₒₘ); 6,96 (m, 1 H, Hₐᵣₒₘ); 6,98-7,12 (m, 22 H, Hₐᵣₒₘ); 7,12-7,19 (m, 10 H, Hₐᵣₒₘ); 7,26-7,33 (m, 5 H, Hₐᵣₒₘ); 7,41-7,48 (m, 4 H, Hₐᵣₒₘ) ppm.
¹³C-NMR (CD₂Cl₂): 20,9; 21,4; 57,4; 95,1 (d, *J*_{CP}= 9,2 Hz); 95,5 (d, *J*_{CP}= 8,8 Hz); 114,2; 121,4 (d, *J*_{CP}= 10,0 Hz); 125,3; 127,2; 127,3; 127,3; 127,3; 127,4; 127,4; 127,5; 127,8; 128,3; 128,8; 129,1 (d, *J*_{CP}= 3,2 Hz); 129,2 (d, *J*_{CP}= 2,9 Hz); 129,5; 130,0; 130,3; 130,7 (d, *J*_{CP}= 3,7 Hz); 131,7 (d, *J*_{CP}= 3,3 Hz); 133,0; 133,5 (d, *J*_{CP}= 14,0 Hz); 138,3 (d, *J*_{CP}= 7,2 Hz); 142,7 (d, *J*_{CP}= 4,3 Hz); 142,9; 143,0 (d, *J*_{CP}= 4,6 Hz); 147,0 (d, *J*_{CP}= 8,8 Hz); 151,1 (d, *J*_{CP}= 2,9 Hz) ppm.

### 2,2'-((3-Methoxy-3',5,5'-trimethyl-[1,1'-biphenyl]-2,2'-diyl)bis(oxy))bis(4,4,5,5-tetraphenyl-1,3,2-dioxaphospholan).

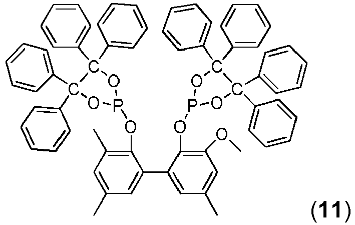

Eine Lösung von 3-Methoxy-3',5,5'-trimethyl-[1,1'-biphenyl]-2,2'-diol (0,320 g; 1,241 mmol) in THF (9 ml) wurde mit zwei Äquivalenten n-Butyllithium, gelöst in Hexan (5 ml), bei -20 °C versetzt. Die Mischung wurde für 20 min bei -20 °C gerührt und dann eine Lösung von 2-Chloro-4,4,5,5-tetraphenyl-1,3,2-dioxaphospholan (1,176 g; 2,729 mmol) in THF (6 ml) bei Raumtemperatur zugegeben. Die Reaktionsmischung wurde über Nacht gerührt und das Lösungsmittel im Vakuum entfernt. Toluol (20 ml) wurde zugegeben und die resultierende Suspension filtriert. Das Lösungsmittel wurde im Vakuum entfernt. Der erhaltene Feststoff wurde bei 50°C / 0,1 mbar getrocknet und dann aus Hexan (27 ml) umkristallisiert. Ausbeute: 1,082 g (1,034 mmol; 83 %).
Elementaranalyse (ber. für C₆₈H₅₆O₇P₂ = 1047,13 g/ mol) C 78,11 (78,00); H 5,38 (5,39); P 6,02 (5,92) %.
³¹P-NMR (CD₂Cl₂): 143,4 (d, *J*_{PP}= 27,2 Hz); 145,1 (d, *J*_{PP}= 27,2 Hz) ppm.
¹H-NMR (CD₂Cl₂): 1,89 (m, 3 H); 2,26 (m, 3 H); 2,40 (m, 3 H); 3,95 (s, 3 H); 6,75 (m, 1 H, Hₐᵣₒₘ); 6,87 (m, 1 H, Hₐᵣₒₘ); 6,94 (m, 1 H, Hₐᵣₒₘ); 6,98 (m, 1 H, Hₐᵣₒₘ); 7,02-7,04 (m, 1 H, Hₐᵣₒₘ); 7,04-7,09 (m, 11 H, Hₐᵣₒₘ); 7,09-7,11 (m, 4 H, Hₐᵣₒₘ); 7,11-7,21 (m, 12 H, Hₐᵣₒₘ); 7,21-7,26 (m, 3 H, Hₐᵣₒₘ); 7,26-7,36 (m, 5 H, Hₐᵣₒₘ); 7,40-7,57 (m, 4 H, Harom) ppm.
¹³C-NMR (CD₂Cl₂): 17,0; 21,0; 21,5; 57,2; 94,8; 114,0; 125,9; 127,3; 127,4; 127,6; 128,3; 128,9; 129,0; 129,1; 129,2; 129,4; 130,0; 130,2; 131,0; 131,2; 132,4 (m); 133,0; 133,6; 138,7 (d, *J*_{CP}= 6,8 Hz); 142,6 (d, *J*_{CP}= 4,0 Hz); 142,7 (m); 143,2; 146,5 (d, *J*_{CP}= 7,7 Hz); 151,4 (d, *J*_{CP}= 3,5 Hz) ppm.

### 2,2'-((3-Methoxy-4',5,5'-trimethyl-[1,1'-biphenyl]-2,2'-diyl)bis(oxy))bis(4,4,5,5-tetraphenyl-1,3,2-dioxaphospholan).

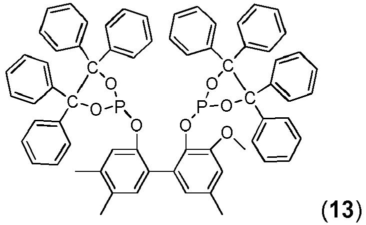

Eine gerührte Lösung von 3-Methoxy-4',5,5'-trimethyl-[1,1'-biphenyl]-2,2'-diol (0,328 g; 1,268 mmol) in Toluol (7 ml) wurde mit Triethylamin (0,402 g; 3,974 mmol) versetzt. Die erhaltene Mischung wurde tropfenweise bei 0 °C zu einer Lösung von 2-Chloro-4,4,5,5-tetraphenyl-1,3,2-dioxaphospholan (1,202 g; 2,789 mmol) in Toluol (10 ml) gegeben und die noch 45 min bei 0 °C gerührt. Dann wurde die Reaktionsmischung drei Tage bei Raumtemperatur und anschließend 37 h bei 70 °C gerührt. Es wurde über Kieselgel filtriert, das Filtrat im Vakuum bis zur Trockne eingeengt und der erhaltene Feststoff für 2 h bei Raumtemperatur im Vakuum getrocknet. Ausbeute: 1,003 g (0,958 mmol; 76 %).
Elementaranalyse (ber. für C₆₈H₅₆O₇P₂ = 1047,07 g/ mol) C 78,13 (78,00); H 5,41 (5,39); P 6,03 (5,92) %.
³¹P-NMR (CD₂Cl₂): 139,7 (d, *J*_{PP}= 15,6 Hz); 145,1 (d, *J*_{PP}= 15,6 Hz) ppm.
¹H-NMR (CD₂Cl₂): 2,18 (s, 3 H); 2,22 (s, 3 H); 2,39 (s, 3 H) 3,95 (s, 3 H); 6,32 (s, 1H, Hₐᵣₒₘ), 6,76 (br, 1 H, Hₐᵣₒₘ); 6,91(br, 1 H, Hₐᵣₒₘ); 6,92 (s, 1 H, Hₐᵣₒₘ); 6,99-7,52 (m, 20 H, Harom) ppm. ¹³C-NMR (CD₂Cl₂): 19,0; 19,6; 21,4; 57,4; 95,0 (d, *J*_{CP}= 8,8 Hz); 95,4 (d, *J*_{CP}= 8,4 Hz); 114,0; 122,7 (d, *J*_{CP}= 9,8 Hz); 125,4; 125,6; 127,0; 129,5; 130,0; 130,3; 131,5; 131,7; 131,9; 133,1; 133,4; 137,3; 138,3; 140,2 (d, *J*_{CP}= 4,0 Hz); 141,0 (d, *J*_{CP}= 5,3 Hz); 142,7 (d, *J*_{CP}= 4,2 Hz); 142,9 (d, *J*_{CP}= 5,8 Hz); 143,0 (d, *J*_{CP}= 4,6 Hz); 146,7 (d, *J*_{CP}= 7,2 Hz); 151,0 (d, *J*_{CP}= 3,4 Hz) ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Die als Substrat eingesetzten Substrate 1-Octen (Aldrich), cis/trans-2-Penten (Aldrich) und n-Octene (Oxeno GmbH, Octenisomerengemisch aus 1-Octen: ∼3 %; *cis*+*trans-*2-Octen; -49%; *cis*+*trans*-3-Octen: ∼29 %; *cis+trans-Octen-*4*:* ∼16 %; gerüstisomere Octene: -3 %) wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien) (OMG AG & Co. KG, Hanau, DE) als Katalysatorvorstufe in Toluol eingefüllt: Für Versuche mit 100 ppm-m Rhodium 10 ml einer 4,31 millimolaren, für 40 ppm-m die gleiche Menge einer entsprechend verdünnte Lösung. Anschließend wurde die entsprechende Menge der in Toluol gelösten Phosphitverbindung, in der Regel 2 bis 5 Ligandäquivalente pro Rhodium, zugemischt. Durch Zugabe von weiterem Toluol (Die Gesamtmasse an Toluol wurde bestimmt für die GC-Analyse, s.u.) wurde das Anfangsvolumen der Katalysatorlösung auf a) 41,0 ml eingestellt bei beabsichtigter Zugabe von 15 ml des Olefins über die Druckpipette (1-Octen, n-Octenen und Versuche mit erhöhter Konzentration an 2-Penten), oder b) 51,9 ml eingestellt bei beabsichtigter Zugabe von 4,1 ml 2-Penten. Die jeweils eingebrachte Masse an Toluol wurde bestimmt. Einwaagen der Olefine: 1-Octen (10,62 g; 94,64 mmol), n-Octene (10,70 g; 95,35 mmol), 2-Penten 9,75 g; 139,00 mmol. Der Autoklav wurde bei einem Gesamtgasdruck (Synthesegas: Linde; H₂ (99,999%) : CO (99,997%) = 1:1) von a) 42 bar für einen Enddruck von 50 bar; b) 12 bar für den Enddruck von 20 bar und c) 7 bar für einen Enddruck von 10 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar, b) 19,5 bar für einen Enddruck von 20 bar und c) 9,5 bar für einen Enddruck von 10 bar erhöht das jeweils in der Tabelle angegebene Olefin(-gemisch) mit einem in der Druckpipette eingestellten Überdruck von ca. 3 bar zugepresst. Die Reaktion wurde bei konstantem Druck von jeweils 50, 20 bzw. 10 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Ergebnisse der Katalyseversuche

Solvens: Toluol
Ausb. = Ausbeute
Sel. = Selektivität
p = Druck in [bar]
T = Temperatur in [°C]
t = Zeit in [h]
[Rh] = Rhodiumkonzentration in [ppm]
L/Rh = Verhältnis von Ligand zum Rhodium

Als Vergleichsliganden wurden die Liganden **A** und **B** ausgewählt. Ihre Herstellung erfolgte nach DE 10 2006 058 682 A1.

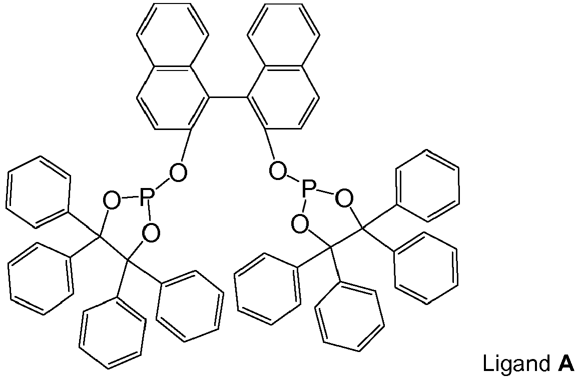

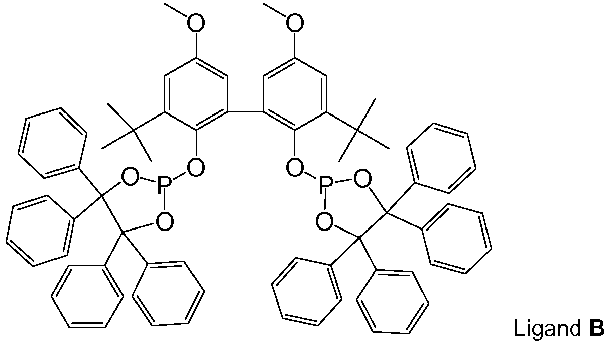

Die erfindungsgemäßen Verbindungen sind mit * gekennzeichnet.

**Tabelle 1: 1-Octen**

| **Ligand** | **p (bar)** | **T (°C)** | **t (h)** | **[Rh] (ppm)** | **L/Rh** | **Ausb. (%)** | **Sel. (%)** |
|---|---|---|---|---|---|---|---|
| **4*** | 50 | 100 | 4 | 40 | 2 | 93 | 90,7 |
| **7*** | 50 | 100 | 4 | 40 | 2 | 92 | 87,3 |
| **9*** | 50 | 100 | 4 | 40 | 2 | 92 | 91,5 |
| **11*** | 50 | 100 | 4 | 40 | 2 | 89 | 95,1 |
| **13*** | 50 | 100 | 4 | 40 | 2 | 97 | 73,9 |
| **A** | 50 | 100 | 4 | 40 | 2 | 89 | 83 |

Wie der Tabelle 1 zu entnehmen ist, zeichnen sich die Liganden in der Hydroformylierung von terminalen Olefinen, hier konkreter 1-Octen, durch sehr gute Ausbeuten aus. Alle erfindungsgemäßen Verbindungen haben eine Ausbeute, welche mindestens so gut ist, wie die des Vergleichsliganden **A.**

Die Verbindung **11,** welche die gleiche Ausbeute wie der Vergleichsligand **A** aufweist, 89 %, hat aber eine deutlich bessere Selektivität.

**Tabelle 2: 2-Penten**

| **Ligand** | **p (bar)** | **T (°C)** | **t (h)** | **[Rh] (ppm)** | **L/Rh** | **Ausb. (%)** | **Sel. (%)** |
|---|---|---|---|---|---|---|---|
| **4*** | 20 | 120 | 4 | 100 | 2 | 69 | 67,9 |
| **7*** | 20 | 120 | 4 | 100 | 2 | 68 | 56,9 |
| **9*** | 20 | 120 | 4 | 100 | 2 | 75 | 68,4 |
| **11*** | 20 | 120 | 4 | 100 | 2 | 47 | 79,5 |
| **13*** | 20 | 120 | 4 | 100 | 2 | 72 | 72,5 |
| **B** | 20 | 120 | 4 | 96 | 2 | 14 | 99 |

Mit dem Vergleichsliganden **B** konnte für 2-Penten zwar eine sehr gute Selektivität, 99 %, erzielt werden, jedoch ist die Ausbeute mit 14 % so gering, dass der Einsatz eines solchen Ligandens für ein großtechnisches Verfahren nur wenig interessant ist. Die Raum-Zeit-Ausbeuten mit diesem Liganden sind so schlecht, sodass dies aus ökonomischer Sicht gegen den Einsatz des Vergleichsliganden **B** spricht.

Die erfindungsgemäßen Verbindungen weißen alle eine annehmbare bis sehr gute Ausbeute in Kombination mit einer guten Selektivität auf.

Wie die Versuchsergebnisse zeigen, wird die gestellte Aufgabe durch die erfindungsgemäßen Verbindungen gelöst.

Es ist somit erstmalig gelungen, Bisphosphite zu generieren, die einen unsymmetrischen Biaryl-Zentral-Baustein, also ein unsymmetrisches Rückgrat, enthalten und gute bis sehr gute Hydroformylierungseigenschaften aufweisen. Dies konnte mit einer Vielzahl von Beispielen belegt werden. Solche konkreten Strukturen und derartige Liganden waren bis dato gänzlich unbekannt und nicht zugänglich.

Diese Bisphosphite weisen eine neuartige Asymmetrie auf. Das besondere hierbei ist die Asymmetrie innerhalb des Biaryl-Zentral-Bausteins, die zu unsymmetrischen Bisphosphiten führt. Diese unsymmetrischen Bisphosphite sind strukturell somit gänzlich von denen im Stand der Technik beschriebenen Bisphoshiten verschieden, bei denen unsymmetrische Bisphosphit-Liganden durch eine bestimmte Anordnung von symmetrischen Biaryl-Bausteinen generiert werden, indem sich beispielsweise die beiden Flügel-Bausteine unterscheiden, die einzelnen Bausteine (Zentral-Baustein und Flügel-Bausteine) an sich aber symmetrisch sind.
Elementaranalyse (ber. für C₄₂H₃₆O₇P₂ = 714,69 g/ mol) C 70,59 (70,58); H 5,28 (5,08); P 8,85 (8,67) %.
³¹P-NMR (CD₂Cl₂): 144,3 (d, *J*_{PP} = 9,1 Hz); 148,1 (d, *J*_{PP} = 9,1 Hz) ppm.
¹H-NMR (CD₂Cl₂): 1,51 (m, 9 H); 2,45 (m, 3 H); 4,06 (s, 3 H); 6,80-6,87 (m, 3 H, Harom); 6,98-7,03 (m, 2 H, Hₐᵣₒₘ); 7,03-7,05 (m, 1 H, Hₐᵣₒₘ); 7,28-7,35 (m, 8 H, Harom); 7,35-7,38 (m, 1 H, Hₐᵣₒₘ); 7,38-7,43 (m, 2 H, Hₐᵣₒₘ); 7,46-7,54 (m, 4 H, Hₐᵣₒₘ) ppm.
¹³C-NMR (CD₂Cl₂): 21,5; 57,0; 113,8; 118,4 (d, *J*_{CP}= 10,1 Hz); 121,8; 122,5 (d, *J*_{CP}= 14,1 Hz); 124,9; 125,5 (d, *J*_{CP}= 17,3 Hz); 127,9; 128,7; 129,4; 129,4 (d, *J*_{CP}= 16,8 Hz); 130,1 (d, *J*_{CP}= 16,1 Hz); 131,3; 131,5; 131,6; 132,0; 134,4; 138,0; 149,5 (d, *J*_{CP}= 4,8 Hz); 149,7 (d, *J*_{CP}= 4,4 Hz); 149,8 (d, *J*_{CP}= 7,0 Hz); 151,2 (d, *J*_{CP}= 3,2 Hz); 153,4 ppm.

### 2,2'-((5'-(tert-Butyl)-3-methoxy-5-methyl-[1,1'-biphenyl]-2,2'-diyl)bis(oxy))bis(4,4,5,5-tetraphenyl-1,3,2-dioxaphospholan).

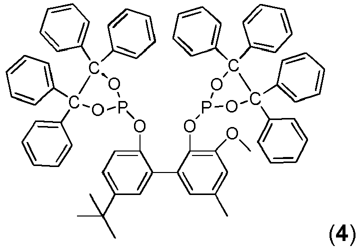

Eine Lösung von 4'-(*tert*-Butyl)-3-methoxy-5-methyl-[1,1'-biphenyl]-2,2'-diol (0,327 g; 1,143 mmol) in THF (5 ml) wurde mit 2 Äquivalenten n-Butyllithium in Hexan (4,3 ml) bei -20 °C versetzt. Die Mischung wurde für 20 min bei -20 °C gerührt, und nach Erwärmen auf Raumtemperatur eine Lösung von 2-Chloro-4,4,5,5-tetraphenyl-1,3,2-dioxaphospholan (0,985 g; 2,287 mmol) in THF (8 ml) zugegeben. Die Reaktionsmischung wurde über Nacht gerührt und das Lösungsmittel im Vakuum abgezogen. Toluol (20 ml) wurde zugegeben und die resultierende Lösung filtriert. Das trübe Filtrat wurde nochmals über Kieselgel filtriert und das Lösungsmittel im Vakuum entfernt. Der erhaltene Feststoff wurde bei 50 °C / 0,1 mbar getrocknet und dann aus heißem Acetonitril (11 ml) umkristallisiert. Ausbeute: 0,840 g (0,781 mmol; 68 %).
Elementaranalyse (ber. für C₇₀H₆₀O₇P₂ = 1075,19 g/ mol) C 78,36 (78,20); H 5,75 (5,62); P 5,95 (5,76) %.
³¹P-NMR (CD₂Cl₂): 139,3 (d, *J*_{PP}= 13,6 Hz); 145,2 (d, *J*_{PP}= 13,6 Hz) ppm.
¹H-NMR (CD₂Cl₂): 1,26 (m, 9 H); 2,38 (m, 3 H); 3,95 (s, 3 H); 6,54 (m, 1 H, Hₐᵣₒₘ); 6,70 (m, 1 H, Hₐᵣₒₘ); 6,91 (m, 1 H, Hₐᵣₒₘ); 6,98-7,04 (m, 4 H, Hₐᵣₒₘ); 7,05-7,13 (m, 20 H, Hₐᵣₒₘ); 7,13-7,21 (m, 10 H, Hₐᵣₒₘ); 7,33-7,40 (m, 4 H, Hₐᵣₒₘ); 7,49-7,55 (m, 4 H, Hₐᵣₒₘ) ppm.
¹³C-NMR (CD₂Cl₂): 31,5; 57,4; 94,9 (d, *J*_{CP}= 9,0 Hz); 95,3 (d, *J*_{CP}= 8,5 Hz); 114,2; 118,9 (d, *J*_{CP}= 9,0 Hz); 121,1; 125,6; 127,2; 127,3; 127,4; 127,5; 127,6; 127,9; 127,9; 129,1 (d, *J*_{CP}= 3,0 Hz); 129,2 (d, *J*_{CP}= 3,0 Hz); 130,0; 130,3; 131,3; 132,0; 133,4; 138,5 (d, *J*_{CP}= 9,0 Hz); 142,6 (d, *J*_{CP}= 4,0 Hz); 142,8; 143,0; 143,0; 148,6 (d, *J*_{CP}= 6,8 Hz); 151,0 (d, *J*_{CP}= 3,4 Hz); 152,6 ppm.

### 6,6'-((4'-(tert-Butyl)-3-Methoxy-5-methyl-[1,1'-biphenyl]-2,2'-diyl)bis(oxy))bis(9-(tert-butyl)-4-methoxy-2-methyldibenzo[d,f][1,3,2]dioxaphosphepin).

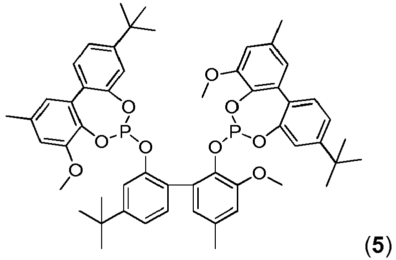

Eine Lösung von 4'-(*tert*-Butyl)-3-methoxy-5-methyl-[1,1'-biphenyl]-2,2'-diol (0,274 g; 0,957 mmol) in THF (10 ml) wurde mit einer Lösung von zwei Äquivalenten n-Butyllithium in Hexan (3,59 ml) bei -20 °C versetzt, die erhaltene Mischung noch 20 min bei dieser Temperatur gerührt und dann eine Lösung von 9-(*tert*-Butyl)-6-chloro-4-methoxy-2-methyldibenzo[*d*,*f*][1,3,2]dioxaphosphepin (0,792 g; 2,258 mmol) in THF (11 ml) bei Raumtemperatur zugegeben. Die Reaktionsmischung wurde über Nacht gerührt und das Lösungsmittel im Vakuum entfernt. Toluol (25 ml) wurde zugegeben und die resultierende Suspension filtriert. Das Filtrat wurde nochmals über Kieselgel filtriert und das Lösungsmittel im Vakuum entfernt. Der erhaltene Feststoff wurde für 3 h bei 50 °C / 0,1 mbar getrocknet. Ausbeute: 0,856 g (0,936 mmol; 98 %).
Elementaranalyse (ber. für C₅₄H₆₀O₉P₂ = 915,01 g/ mol) C 70,67 (70,88); H 6,52 (6,61); P 6,69 (6,77) %.
³¹P-NMR (CD₂Cl₂): 141,9 (d, *J*_{PP}= 7.8 Hz); 142,2 (d, *J*_{PP}= 7.8 Hz); 145,1 (d, *J*_{PP}= 7.8 Hz); 145,2 (d, *J*_{PP}= 7.8 Hz) ppm.
¹H-NMR (CD₂Cl₂): 1,22-1,33 (dd, 18 H); 1,37 (m, 9 H); 2,42 (m, 9 H); 3,81-3,88 (dd, 6 H); 4,02 (s, 3 H); 6,79-6,85 (m, 3 H, Hₐᵣₒₘ); 6,88 (m, 2 H, Hₐᵣₒₘ); 6,90-6,98 (m, 1 H, Hₐᵣₒₘ); 6,95 (m, 1 H, Hₐᵣₒₘ); 7,00-7,05 (m, 1 H, Harom); 7,20-7,35 (m, 4 H, Hₐᵣₒₘ); 7,37-7,44 (m, 3 H, Hₐᵣₒₘ) ppm.

### 6,6'-((5'-Isopropyl-3-methoxy-4',5-dimethyl-[1,1'-biphenyl]-2,2'-diyl)bis(oxy))didibenzo[d,f][1,3,2]dioxaphosphepin.

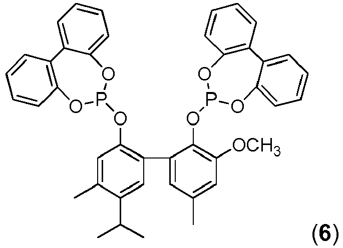

Eine Lösung von 5'-Isopropyl-3-methoxy-4',5-dimethyl-[1,1'-biphenyl]-2,2'-diol (0,485 g; 1,692 mmol) in Toluol (12 ml) wurde mit Pyridin (0,385 g; 3,807 mmol) versetzt und die erhaltene Mischung tropfenweise zu einer auf 3 °C gekühlten Lösung von 6-Chlorodibenzo[*d,f*][1,3,2]dioxaphosphepin (0,933 g; 3,722 mmol) in Toluol (12 ml) gegeben. Die Reaktionsmischung wurde über Nacht gerührt und dann filtriert. Das Filtrat wurde im Vakuum bis zur Trockne eingedampft und der erhaltene Feststoff bei 50 °C /0,1 mbar getrocknet. Die zurückgebliebene Substanz wurde durch Säulenchromatografie gereinigt (Hexan/Toluol, 1:4, R*_{f}* = 0,4). Ausbeute: 0,725 g (1,015 mmol; 60 %).
Elementaranalyse (ber. für C₄₂H₃₆O₇P₂ = 714,69 g/ mol) C 70,77 (70,58); H 5,28 (4,98); P 8,85 (8,86) %.
³¹P-NMR (CD₂Cl₂): 145,4 (d, *J*_{PP} = 7,2 Hz); 148,2 (d, *J*_{PP} = 7,2 Hz) ppm.
¹H-NMR (CD₂Cl₂): 1,24 (s, 3 H); 1,26 (s, 3 H); 2,43 (m, 3 H); 2,51 (m, 3 H); 3,22 (m, 1 H); 4,03 (s, 3 H); 6,72-6,83 (m, br, 3 H, Hₐᵣₒₘ); 6,95-6,99 (m, 2 H, Harom); 6,99-7,02 (m, 1 H, Hₐᵣₒₘ); 7,13 (m, 1 H, Hₐᵣₒₘ); 7,22-7,33 (m, 9 H, Hₐᵣₒₘ); 7,42-7,50 (m, 4 H, Hₐᵣₒₘ) ppm.
¹³C-NMR (CD₂Cl₂): 19,3; 21,5; 23,5; 29,4; 57,0; 113,8; 122,6 (d, *J*_{CP}= 14,8 Hz); 124,8; 125,5 (d, *J*_{CP}= 18,7 Hz); 128,3; 128,8; 129,4 (d, *J*_{CP}= 14,5 Hz); 130,0 (d, *J*_{CP}= 16,3 Hz); 131,5; 131,6; 134,3; 136,8; 137,9 (d, *J*_{CP}= 6,3 Hz); 143,4; 147,5 (d, *J*_{CP}= 8,2 Hz); 149,6 (d, *J*_{CP}= 5,0 Hz); 149,7 (d, *J*_{CP}= 4,5 Hz); 151,0 (d, *J*_{CP}= 2,8 Hz) ppm.

### 2,2'-((5'-Isopropyl-3-methoxy-4',5-dimethyl-[1,1'-biphenyl]-2,2'-diyl)bis(oxy))bis(4,4,5,5-tetraphenyl-1,3,2-dioxaphospholan).

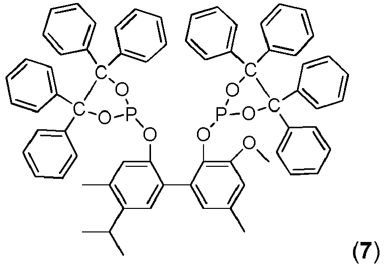

Eine Lösung von 5'-Isopropyl-3-methoxy-4',5-dimethyl-[1,1'-biphenyl]-2,2'-diol (0,330 g; 1,153 mmol) in THF (5 ml) wurde mit 2 Äquivalenten n-Butyllithium, gelöst in Hexan (4,3 ml), bei - 20°C versetzt. Die Mischung wurde für 20 min bei -20 °C gerührt, und nach Erwärmen auf Raumtemperatur eine Lösung von 2-Chloro-4,4,5,5-tetraphenyl-1,3,2-dioxaphospholan (0,994 g; 2,307 mmol) in THF (8 ml) zugegeben. Die Reaktionsmischung wurde über Nacht gerührt und das Lösungsmittel im Vakuum entfernt. Toluol (20 ml) wurde zugegeben und die resultierende Lösung über Kieselgel filtriert. Das Filtrat wurde im Vakuum bis zur Trockne eingeengt. Der erhaltene Feststoff wurde bei 50 °C / 0,1 mbar getrocknet und dann aus heißem Acetonitril (12 ml) umkristallisiert. Ausbeute: 0,782 g (0,728 mmol; 63 %).
Elementaranalyse (ber. für C₇₆H₆₆O₇P₂ = 1075,19 g/ mol) C 78,18 (78,20); H 5,69 (5,62); P 5,87 (5,76) %.
³¹P-NMR (CD₂Cl₂): 139,3 (d, *J*_{PP}= 9,1 Hz); 145,0 (d, *J*_{PP}= 9,1 Hz) ppm.
¹H-NMR (CD₂Cl₂): 1,21 (m, 6 H); 2,27 (m, 3 H); 2,42 (m, 3 H); 3,96 (s, 3 H); 6,07 (m, 1 H, Hₐᵣₒₘ); 6,68 (m, 1 H, Hₐᵣₒₘ); 6,93 (m, 3 H, Hₐᵣₒₘ); 6,95-7,05 (m, 7 H, Hₐᵣₒₘ); 7,05-7,16 (m, 18 H, Hₐᵣₒₘ); 7,16-7,23 (m, 7 H, Hₐᵣₒₘ); 7,30-7,38 (m, 4 H, Hₐᵣₒₘ); 7,49-7,56 (m, 4 H, Hₐᵣₒₘ) ppm.
¹³C-NMR (CD₂Cl₂): 19,1; 21,5; 23,5; 29,3; 57,5; 94,8 (d, *J*_{CP}= 8,8 Hz); 95,5 (d, *J*_{CP}= 8,2 Hz); 114,2; 123,3 (d, *J*_{CP}= 10,0 Hz); 125,4; 127,1; 127,2; 127,3; 127,3; 127,4; 127,6; 128,3; 128,7 (d, *J*_{CP}= 3,4 Hz); 129,0; 129,3; 129,9; 130,4 (m); 132,2 (d, *J*_{CP}= 3,0 Hz); 133,5; 136,1; 138,5 (d, *J*_{CP}= 6,8 Hz); 142,5; 142,7; 142,8; 143,0; 143,1 (d, *J*_{CP}= 4,6 Hz); 146,1 (d, *J*_{CP}= 5,2 Hz); 151,0 (d, *J*_{CP}= 3,6 Hz) ppm.

### 6,6'-((3-Methoxy-5,5'-dimethyl-[1,1'-biphenyl]-2,2'-diyl)bis(oxy))didibenzo[d,f][1,3,2]dioxaphosphepin.

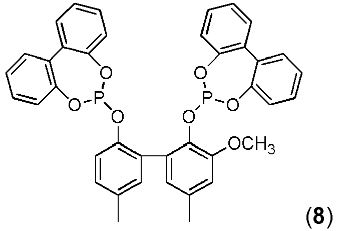

Eine Lösung von 3-Methoxy-5,5'-dimethyl-[1,1'-biphenyl]-2,2'-diol (0,419 g; 1,714 mmol) in Toluol (12 ml) wurde mit Pyridin (0,390 g; 3,857 mmol) versetzt und die erhaltene Mischung bei 3 °C tropfenweise zu einer Lösung von 6-Chlorodibenzo[*d,f*][1,3,2]dioxaphosphepin (0,945 g; 3,771 mmol) in Toluol (12 ml) gegeben. Die Reaktionsmischung wurde über Nacht gerührt und dann filtriert. Das Filtrat wurde im Vakkum bis zur Trockne eingeengt und der erhaltene Feststoff bei 50 °C getrocknet. Die Substanz wurde in heißem Acetonitril gelöst (25 ml). Kristallisation erfolgt nach Abkühlen der Lösung und Einengen im Vakuum auf die Hälfte des Volumens. Ausbeute: 0,205 g (0,030 mmol; 18 %).
Elementaranalyse (ber. für C₃₉H₃₀O₇P₂ = 672,61 g/ mol) C 69,58 (69,64); H 4,30 (4,49); P 9,25 (9,21) %.
³¹P-NMR (CD₂Cl₂): 145,6 (d, *J*_{PP} = 8,4 Hz); 148,2 (d, *J*_{PP} = 8,4 Hz) ppm.
¹H-NMR (CD₂Cl₂): 42 (m, 6 H); 4,05 (s, 3 H); 6,75-6,82 (m, 3 H, Hₐᵣₒₘ); 6,95-7,02 (m, 3 H, Hₐᵣₒₘ); 7,17 (m, 1 H, Hₐᵣₒₘ); 7,24-7,36 (m, 10 H, Hₐᵣₒₘ); 7,42-7,50 (m, 4 H, Hₐᵣₒₘ) ppm.
¹³C-NMR (CD₂Cl₂): 20,9; 21,4; 57,0; 113,9; 120,9 (d, *J*_{CP}= 10,3 Hz); 122,6 (d, *J*_{CP}= 11,6 Hz); 124,7; 125,5 (d, *J*_{CP}= 19,2 Hz); 129,4 (d, *J*_{CP}= 9,9 Hz); 129,8; 129,9; 130,1; 130,5 (d, *J*_{CP}= 3,3 Hz); 131,2 (d, *J*_{CP}= 3,0 Hz); 131,5 (m); 133,0; 134,3 (d, *J*_{CP}= 4,6 Hz); 147,8 (d, *J*_{CP}= 8,4 Hz); 149,4 (d, *J*_{CP}= 5,0 Hz); 149,7 (d, *J*_{CP}= 4,6 Hz); 151,0 (d, *J*_{CP}= 2,9 Hz) ppm.

### 2,2'-((3-Methoxy-5,5'-dimethyl-[1,1'-biphenyl]-2,2'-diyl)bis(oxy))bis(4,4,5,5-tetraphenyl-1,3,2-dioxaphospholan).

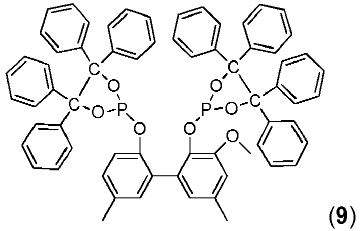

Eine Lösung von 3-Methoxy-5,5'-dimethyl-[1,1'-biphenyl]-2,2'-diol (0,241 g; 0,986 mmol) in THF (4 ml) wurde mit 2 Äquivalenten n-Butyllithium, gelöst in Hexan (3,7 ml), bei -20 °C versetzt. Die Mischung wurde zunächst für 20 min bei -20 °C gerührt und nach Erwärmen auf Raumtemperatur eine Lösung von 2-Chloro-4,4,5,5-tetraphenyl-1,3,2-dioxaphospholan (0,850 g; 1,973 mmol) in THF (7 ml) zugegeben. Die Reaktionsmischung wurde über Nacht gerührt und das Lösungsmittel im Vakuum eingeengt. Der Rückstand wurde mit Toluol (20 ml) verrührt und die resultierende Suspension über Kieselgel filtriert. Das Filtrat wurde im Vakkum zur Trockne eingeengt. Der erhaltene Feststoff wurde bei 50 °C / 0,1 mbar getrocknet und dann aus heißem Acetonitril (11 ml) umkristallisiert. Die zurückgebliebene Substanz wurde durch Säulenchromatografie gereinigt (Hexan/Toluol, 1:2, R*_{f}* = 0,3). Ausbeute: 0,430 g (0,416 mmol; 43 %).
Elementaranalyse (ber. für C₆₇H₅₄O₇P₂ = 1033,10 g/ mol) C 77,64 (77,89); H 5,50 (5,27); P 6,05 (6,00) %.
³¹P-NMR (CD₂Cl₂): 139,9 (d, *J*_{PP}= 17,5 Hz); 145,1 (d, *J*_{PP}= 17,5 Hz) ppm.
¹H-NMR (CD₂Cl₂): 26 (m, 3 H); 2,35 (m, 3 H); 3,91 (s, 3 H); 6,57 (m, 1 H, Hₐᵣₒₘ); 6,63 (m, 1 H, Hₐᵣₒₘ); 6,88 (m, 1 H, Hₐᵣₒₘ); 6,96 (m, 1 H, Hₐᵣₒₘ); 6,98-7,12 (m, 22 H, Hₐᵣₒₘ); 7,12-7,19 (m, 10 H, Hₐᵣₒₘ); 7,26-7,33 (m, 5 H, Hₐᵣₒₘ); 7,41-7,48 (m, 4 H, Hₐᵣₒₘ) ppm.
¹³C-NMR (CD₂Cl₂): 20,9; 21,4; 57,4; 95,1 (d, *J*_{CP}= 9,2 Hz); 95,5 (d, *J*_{CP}= 8,8 Hz); 114,2; 121,4 (d, *J*_{CP}= 10,0 Hz); 125,3; 127,2; 127,3; 127,3; 127,3; 127,4; 127,4; 127,5; 127,8; 128,3; 128,8; 129,1 (d, *J*_{CP}= 3,2 Hz); 129,2 (d, *J*_{CP}= 2,9 Hz); 129,5; 130,0; 130,3; 130,7 (d, *J*_{CP}= 3,7 Hz); 131,7 (d, *J*_{CP}= 3,3 Hz); 133,0; 133,5 (d, *J*_{CP}= 14,0 Hz); 138,3 (d, *J*_{CP}= 7,2 Hz); 142,7 (d, *J*_{CP}= 4,3 Hz); 142,9; 143,0 (d, *J*_{CP}= 4,6 Hz); 147,0 (d, *J*_{CP}= 8,8 Hz); 151,1 (d, *J*_{CP}= 2,9 Hz) ppm.

### 6,6'-((3-Methoxy-3',5,5'-trimethyl-[1,1'-biphenyl]-2,2'-diyl)bis(oxy))didibenzo[d,f][1,3,2]dioxaphosphepin.

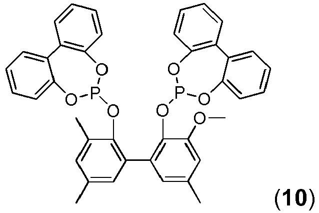

Eine Lösung von 4',5-Dimethoxy-6'-methyl-[1,1'-biphenyl]-2,3'-diol (0,361 g; 2,439 mmol) in Toluol (8 ml) wurde mit Triethylamin (0,444 g; 4,385 mmol) versetzt und auf 0 °C gekühlt. Zu dieser Mischung wurde eine Lösung von 6-Chlorodibenzo[*d*,*f*][1,3,2]dioxaphosphepin (0,57 g; 3,419 mmol) in Toluol (13 ml) tropfenweise zugegeben. Die Reaktionsmischung wurde für 45 min bei 0 °C und über Nacht bei Raumtemperatur gerührt, über Kieselgel filtriert und das Filtrat im Vakuum wurde bis zur Trockne eingeengt. Ausbeute: 0,666 g (0,696 mmol; 69 %).
Elementaranalyse (ber. für C₄₀H₃₂O₇P₂ = 686,16 g/ mol): C 69,75 (69,97); H 4,76 (4,70); P 8,80 (9,02) %.
³¹P-NMR (CD₂Cl₂): 142,4 (d, *J*_{PP}= 9,7 Hz); 144,8 (d, *J*_{PP}= 9,7 Hz); 146,7 (d, *J*_{PP}= 13,6 Hz); 148,4 (d, *J*_{PP}= 13,6 Hz) ppm. ¹H-NMR (CD₂Cl₂): 2,01; 2,37; 2,41; 2,42; 2,46; 2,50 (6s, 9 H); 3,90; 4,04 (2s, 3 H); 6,76-7,63 (m, 20 H) ppm.

### 2,2'-((3-Methoxy-3',5,5'-trimethyl-[1,1'-biphenyl]-2,2'-diyl)bis(oxy))bis(4,4,5,5-tetraphenyl-1,3,2-dioxaphospholan).

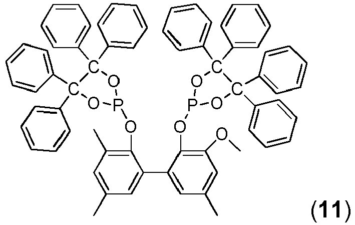

Eine Lösung von 3-Methoxy-3',5,5'-trimethyl-[1,1'-biphenyl]-2,2'-diol (0,320 g; 1,241 mmol) in THF (9 ml) wurde mit zwei Äquivalenten n-Butyllithium, gelöst in Hexan (5 ml), bei -20 °C versetzt. Die Mischung wurde für 20 min bei -20 °C gerührt und dann eine Lösung von 2-Chloro-4,4,5,5-tetraphenyl-1,3,2-dioxaphospholan (1,176 g; 2,729 mmol) in THF (6 ml) bei Raumtemperatur zugegeben. Die Reaktionsmischung wurde über Nacht gerührt und das Lösungsmittel im Vakuum entfernt. Toluol (20 ml) wurde zugegeben und die resultierende Suspension filtriert. Das Lösungsmittel wurde im Vakuum entfernt. Der erhaltene Feststoff wurde bei 50°C / 0,1 mbar getrocknet und dann aus Hexan (27 ml) umkristallisiert. Ausbeute: 1,082 g (1,034 mmol; 83 %).
Elementaranalyse (ber. für C₆₈H₅₆O₇P₂ = 1047,13 g/ mol) C 78,11 (78,00); H 5,38 (5,39); P 6,02 (5,92) %.
³¹P-NMR (CD₂Cl₂): 143,4 (d, *J*_{PP}= 27,2 Hz); 145,1 (d, *J*_{PP}= 27,2 Hz) ppm.
¹H-NMR (CD₂Cl₂): 1,89 (m, 3 H); 2,26 (m, 3 H); 2,40 (m, 3 H); 3,95 (s, 3 H); 6,75 (m, 1 H, Hₐᵣₒₘ); 6,87 (m, 1 H, Hₐᵣₒₘ); 6,94 (m, 1 H, Hₐᵣₒₘ); 6,98 (m, 1 H, Hₐᵣₒₘ); 7,02-7,04 (m, 1 H, Hₐᵣₒₘ); 7,04-7,09 (m, 11 H, Hₐᵣₒₘ); 7,09-7,11 (m, 4 H, Hₐᵣₒₘ); 7,11-7,21 (m, 12 H, Hₐᵣₒₘ); 7,21-7,26 (m, 3 H, Hₐᵣₒₘ); 7,26-7,36 (m, 5 H, Hₐᵣₒₘ); 7,40-7,57 (m, 4 H, Hₐᵣₒₘ) ppm.
¹³C-NMR (CD₂Cl₂): 17,0; 21,0; 21,5; 57,2; 94,8; 114,0; 125,9; 127,3; 127,4; 127,6; 128,3; 128,9; 129,0; 129,1; 129,2; 129,4; 130,0; 130,2; 131,0; 131,2; 132,4 (m); 133,0; 133,6; 138,7 (d, *J*_{CP}= 6,8 Hz); 142,6 (d, *J*_{CP}= 4,0 Hz); 142,7 (m); 143,2; 146,5 (d, *J*_{CP}= 7,7 Hz); 151,4 (d, *J*_{CP}= 3,5 Hz) ppm.

### 6,6'-((3-Methoxy-4',5,5'-trimethyl-[1,1'-biphenyl]-2,2'-diyl)bis(oxy))didibenzo[d,f][1,3,2]dioxaphosphepin.

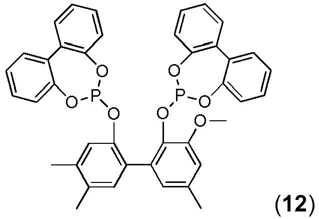

Eine Lösung von 3-Methoxy-4',5,5'-trimethyl-[1,1'-biphenyl]-2,2'-diol (0,334 g; 1,294 mmol) in Toluol (7 ml) wurde mit Triethylamin (0,410 g; 4,048 mmol) versetzt, auf 0 °C gekühlt und anschließend zu dieser Mischung eine Lösung von 6-Chlorodibenzo[*d,f*][1,3,2]dioxaphosphepin (0,791 g; 3,157 mmol) in Toluol (12 ml) tropfenweise zugegeben. Die Reaktionsmischung wurde für 45 min bei 0 °C und drei Tage bei Raumtemperatur gerührt. Die beige-gelbe Suspension wurde filtriert und der erhaltene Feststoff mit ein wenig eiskaltem Toluol gewaschen. Der Feststoff wurde in 40 ml Toluol aufgenommen. Die erhaltene Suspension wurde für 2 h bei 40 °C gerührt und dann warm filtriert. Das Filtrat wurde im Vakuum bis zur Trockne eingeengt und der Rückstand bei 0,1 mbar getrocknet. Ausbeute: 0,254 g (0,370 mmol; 29 %).
Elementaranalyse (ber. für C₄₀H₃₂O₇P₂ = 686,60 g/ mol) C 69,79 (69,97); H 4,81 (4,70); P 9,01 (9,02) %.
³¹P-NMR (CD₂Cl₂): 145,6 (d, *J*_{PP}= 8,4 Hz); 148,2 (d, *J*_{PP}= 8,4 Hz) ppm.
¹H-NMR (CD₂Cl₂): 2,31 (m, 3 H); 2,42 (m, 3 H); 2,43 (m, 3 H); 4,03 (s, 3 H); 6,75-6,81 (m, 3 H, Hₐᵣₒₘ); 6,94-6,98 (m, 2 H, Hₐᵣₒₘ); 7,00 (m, 1 H, Hₐᵣₒₘ); 7,12 (m, 2 H, Hₐᵣₒₘ); 7,23-7,35 (m, 8 H, Hₐᵣₒₘ); 7,42-7,50 (m, 4 H, Hₐᵣₒₘ) ppm.
¹³C-NMR (CD₂Cl₂): 19,2; 19,8; 21,4; 57,0; 113,7; 122,2 (d, *J*_{CP}= 10,1 Hz); 122,5 (d, *J*_{CP}= 13,1 Hz); 124,8; 125,3; 125,6; 125,6; 125,6; 127,9; (d, *J*_{CP}= 3,4 Hz); 128,6; 129,2 (d, *J*_{CP}= 15,5 Hz); 129,3; 129,8 (d, *J*_{CP}= 16,6 Hz); 131,2; 131,5 (m); 133,0; 133,2; 134,3; 137,9; 138,1; 147,8 (d, *J*_{CP}= 8,5 Hz); 149,5 (d, *J*_{CP}= 4,9 Hz); 149,7 (d, *J*_{CP}= 10,1 Hz); 150,9 (d, *J*_{CP}= 2,8 Hz) ppm.

### 2,2'-((3-Methoxy-4',5,5'-trimethyl-[1,1'-biphenyl]-2,2'-diyl)bis(oxy))bis(4,4,5,5-tetraphenyl-1,3,2-dioxaphospholan).

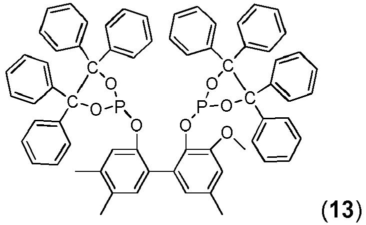

Eine gerührte Lösung von 3-Methoxy-4',5,5'-trimethyl-[1,1'-biphenyl]-2,2'-diol (0,328 g; 1,268 mmol) in Toluol (7 ml) wurde mit Triethylamin (0,402 g; 3,974 mmol) versetzt. Die erhaltene Mischung wurde tropfenweise bei 0 °C zu einer Lösung von 2-Chloro-4,4,5,5-tetraphenyl-1,3,2-dioxaphospholan (1,202 g; 2,789 mmol) in Toluol (10 ml) gegeben und die noch 45 min bei 0 °C gerührt. Dann wurde die Reaktionsmischung drei Tage bei Raumtemperatur und anschließend 37 h bei 70 °C gerührt. Es wurde über Kieselgel filtriert, das Filtrat im Vakuum bis zur Trockne eingeengt und der erhaltene Feststoff für 2 h bei Raumtemperatur im Vakuum getrocknet. Ausbeute: 1,003 g (0,958 mmol; 76 %).
Elementaranalyse (ber. für C₆₈H₅₆O₇P₂ = 1047,07 g/ mol) C 78,13 (78,00); H 5,41 (5,39); P 6,03 (5,92) %.
³¹P-NMR (CD₂Cl₂): 139,7 (d, *J*_{PP}= 15,6 Hz); 145,1 (d, *J*_{PP}= 15,6 Hz) ppm.
¹H-NMR (CD₂Cl₂): 2,18 (s, 3 H); 2,22 (s, 3 H); 2,39 (s, 3 H) 3,95 (s, 3 H); 6,32 (s, 1H, Hₐᵣₒₘ), 6,76 (br, 1 H, Hₐᵣₒₘ); 6,91 (br, 1 H, Hₐᵣₒₘ); 6,92 (s, 1 H, Hₐᵣₒₘ); 6,99-7,52 (m, 20 H, Hₐᵣₒₘ) ppm.
¹³C-NMR (CD₂Cl₂): 19,0; 19,6; 21,4; 57,4; 95,0 (d, *J*_{CP}= 8,8 Hz); 95,4 (d, *J*_{CP}= 8,4 Hz); 114,0; 122,7 (d, *J*_{CP}= 9,8 Hz); 125,4; 125,6; 127,0; 129,5; 130,0; 130,3; 131,5; 131,7; 131,9; 133,1; 133,4; 137,3; 138,3; 140,2 (d, *J*_{CP}= 4,0 Hz); 141,0 (d, *J*_{CP}= 5,3 Hz); 142,7 (d, *J*_{CP}= 4,2 Hz); 142,9 (d, *J*_{CP}= 5,8 Hz); 143,0 (d, *J*_{CP}= 4,6 Hz); 146,7 (d, *J*_{CP}= 7,2 Hz); 151,0 (d, *J*_{CP}= 3,4 Hz) ppm.

### 6-((1-(3-(tert-Butyl)-2-(dibenzo[d,f][1,3,2]dioxaphosphepin-6-yloxy)-5-methoxyphenyl)naphthalen-2-yl)oxy)dibenzo[d,f][1,3,2]dioxaphosphepin.

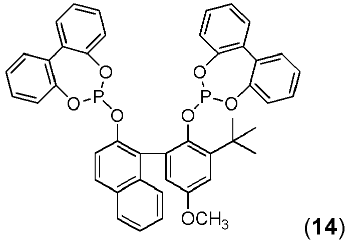

Eine Lösung von 1-(3-(*tert*-Butyl)-2-hydroxy-5-methoxyphenyl)naphthalin-2-ol (0,400 g; 1,240 mmol) in Toluol (7 ml) wurde mit Triethylamin (0,524 g; 5,182 mmol) versetzt und die erhaltene Mischung tropfenweise bei 0 °C zu einer Lösung von 6-Chlorodibenzo[*d,f*][1,3,2]dioxaphosphepin (0,684 g; 2,728 mmol) in Toluol (9 ml) gegeben. Die Reaktionsmischung wurde für 45 min bei 0 °C und über Nacht bei Raumtemperatur gerührt. Die orange-gelbe Mischung wurde über Kieselgel filtriert und das Filtrat im Vakuum bis zur Trockne eingeengt. Der erhaltene Feststoff wurde für 3 h bei 50 °C / 0,1 mbar getrocknet. Ausbeute: 0,503 g (0,670 mmol; 65 %).
Elementaranalyse (ber. für C₄₅H₃₆O₇P₂ = 750,68 g/ mol) C 71,94 (71,99); H 5,01 (4,83); P 8,33 (8,25) %.
³¹P-NMR (CD₂Cl₂): 144,6 (d, *J*_{PP}= 7,8 Hz); 145,9; 146,0; 146,4 (d, *J*_{PP}= 7,8 Hz) ppm.
¹H-NMR (CD₂Cl₂): 1,47+1,60 (2s, 9 H); 3,80+3,82 (2s, 3 H); 6,29 (m, 1H, Harom); 6,70 (m, 1H, Harom); 6,77-8,10 (m, 22H, Hₐᵣₒₘ) ppm.
¹³C-NMR (CD₂Cl₂): 30,8; 31,3; 31,3; 35,7; 35,9; 56,0; 56,0; 114,7; 114,8; 114,9; 115,5; 121,0; 133,0; 134,8; 138,4; 142,0 (d, *J*_{CP}= 2,2 Hz); 144,0; 144,2 (d, *J*_{CP}= 2,8 Hz); 146,6 (d, *J*_{CP}= 4,6 Hz); 148,8; 149,7; 149,9 (d, *J*_{CP}= 2,5 Hz); 150,0 (d, *J*_{CP}= 3,3 Hz); 155,9 (d, *J*_{CP}= 4,0 Hz) ppm.

### 2-(2-(tert-Butyl)-4-methoxy-6-(2-((4,4,5,5-tetraphenyl-1,3,2-dioxaphospholan-2-yl)oxy)naphthalen-1-yl)phenoxy)-4,4,5,5-tetraphenyl-1,3,2-dioxaphospholan.

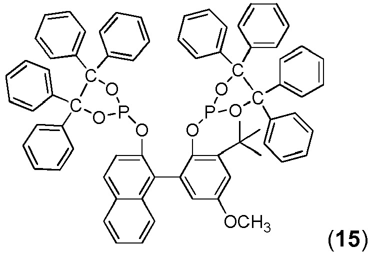

Eine Lösung von 1-(3-(*tert*-Butyl)-2-hydroxy-5-methoxyphenyl)naphthalin-2-ol (0,312 g; 0,969 mmol) in Toluol (6 ml) wurde mit Triethylamin (0,307 g; 3,037 mmol) versetzt und die erhaltene Mischung tropfenweise bei 0 °C zu einer Lösung von 2-Chloro-4,4,5,5-tetraphenyl-1,3,2-dioxaphospholan (0,918 g; 2,131 mmol) in Toluol (10 ml) gegeben. Es wurde 45 min bei 0 °C, 3 Tage bei Raumtemperatur und 37 h bei 70 °C gerührt. Danach wurde die Mischung über Kieselgel filtriert. Das Filtrat wurde bis zur Trockne eingedampft und der erhaltene Feststoff für 2 h bei Raumtemperatur bei 0,1 mbar getrocknet. Ausbeute: 0,891 g (0,802 mmol; 83 %). Das Produkt hält 86% der Signalintensität im ³¹P-NMR Spektrum. Reinigungsversuche waren erfolglos.
Elementaranalyse (ber. für C₇₃H₆₆O₇P₂ = 1111,151 g/ mol): C 73,39 (78,90); H 5,94 (5,44); P 5,26 (5,58) %.
³¹P-NMR (CD₂Cl₂): 140,5 (d, *J*_{PP}= 35,0 Hz); 145,9 (d, *J*_{PP}= 35,0 Hz) ppm.
¹H-NMR (CD₂Cl₂): 1,12 (s, 3H), 3.77 (s, 3H), 6.55-6.06 (m) ppm.
¹³C-NMR (CD₂Cl₂): 30,4; 35,0; 94,2 (d, *J*_{CP}= 7,9 Hz); 94,5 (d, *J*_{CP}= 8,1 Hz); 114,1; 114,7; 121,9 (d, *J*_{CP}= 11,5 Hz); 125,1; 126,9 (d, *J*_{CP}= 6,3Hz); 127,0 (d, *J*_{CP}= 4,2 Hz); 127,1; 127,3; 127,4; 128,0; 128,3 (d, *J*_{CP}= 3,7 Hz); 128,4 (d, *J*_{CP}= 4,4 Hz);128,7; 129,1; 129,6, 130,2; 130,4; 131,4; 142,6 (m); 155,5 ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Die als Substrat eingesetzten Substrate 1-Octen (Aldrich), cis/trans-2-Penten (Aldrich) und n-Octene (Oxeno GmbH, Octenisomerengemisch aus 1-Octen: ∼3 %; *cis*+*trans*-2-Octen; -49%; *cis*+*trans*-3-Octen: -29 %; *cis*+*trans*-Octen-4: -16 %; gerüstisomere Octene: -3 %) wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien) (OMG AG & Co. KG, Hanau, DE) als Katalysatorvorstufe in Toluol eingefüllt: Für Versuche mit 100 ppm-m Rhodium 10 ml einer 4,31 millimolaren, für 40 ppm-m die gleiche Menge einer entsprechend verdünnte Lösung. Anschließend wurde die entsprechende Menge der in Toluol gelösten Phosphitverbindung, in der Regel 2 bis 5 Ligandäquivalente pro Rhodium, zugemischt. Durch Zugabe von weiterem Toluol (Die Gesamtmasse an Toluol wurde bestimmt für die GC-Analyse, s.u.) wurde das Anfangsvolumen der Katalysatorlösung auf a) 41,0 ml eingestellt bei beabsichtigter Zugabe von 15 ml des Olefins über die Druckpipette (1-Octen, n-Octenen und Versuche mit erhöhter Konzentration an 2-Penten), oder b) 51,9 ml eingestellt bei beabsichtigter Zugabe von 4,1 ml 2-Penten. Die jeweils eingebrachte Masse an Toluol wurde bestimmt. Einwaagen der Olefine: 1-Octen (10,62 g; 94,64 mmol), n-Octene (10,70 g; 95,35 mmol), 2-Penten 9,75 g; 139,00 mmol. Der Autoklav wurde bei einem Gesamtgasdruck (Synthesegas: Linde; H₂ (99,999%) : CO (99,997%) = 1:1) von a) 42 bar für einen Enddruck von 50 bar; b) 12 bar für den Enddruck von 20 bar und c) 7 bar für einen Enddruck von 10 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar, b) 19,5 bar für einen Enddruck von 20 bar und c) 9,5 bar für einen Enddruck von 10 bar erhöht das jeweils in der Tabelle angegebene Olefin(-gemisch) mit einem in der Druckpipette eingestellten Überdruck von ca. 3 bar zugepresst. Die Reaktion wurde bei konstantem Druck von jeweils 50, 20 bzw. 10 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Ergebnisse der Katalyseversuche

Solvens: Toluol
Ausb. = Ausbeute
Sel. = Selektivität
p = Druck in [bar]
T = Temperatur in [°C]
t = Zeit in [h]
[Rh] = Rhodiumkonzentration in [ppm]
L/Rh = Verhältnis von Ligand zum Rhodium

Als Vergleichsliganden wurden die Liganden **A** und **B** ausgewählt. Ihre Herstellung erfolgte nach DE 10 2006 058 682 A1.

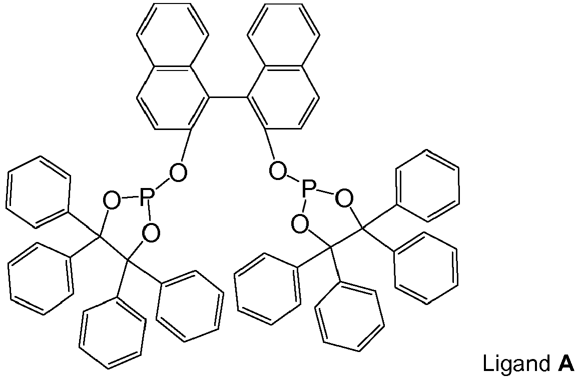

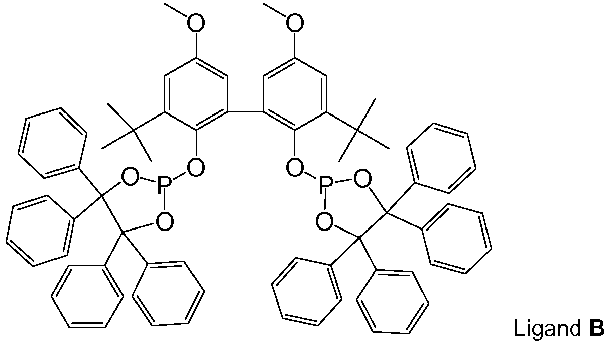

Die erfindungsgemäßen Verbindungen sind mit * gekennzeichnet.

**Tabelle 1: 1-Octen**

| **Ligand** | **p (bar)** | **T (°C)** | **t (h)** | **[Rh] (ppm)** | **L/Rh** | **Ausb. (%)** | **Sel. (%)** |
|---|---|---|---|---|---|---|---|
| **1*** | 50 | 100 | 4 | 40 | 2 | 92 | 93,1 |
| **2*** | 50 | 100 | 4 | 40 | 2 | 91 | 90,5 |
| **3*** | 50 | 100 | 4 | 40 | 2 | 90 | 90,0 |
| **4*** | 50 | 100 | 4 | 40 | 2 | 93 | 90,7 |
| **5*** | 50 | 100 | 4 | 40 | 2 | 91 | 87,3 |
| **6*** | 50 | 100 | 4 | 40 | 2 | 91 | 90,8 |
| **7*** | 50 | 100 | 4 | 40 | 2 | 92 | 87,3 |
| **8*** | 50 | 100 | 4 | 40 | 2 | 93 | 90,2 |
| **9*** | 50 | 100 | 4 | 40 | 2 | 92 | 91,5 |
| **10*** | 50 | 100 | 4 | 40 | 2 | 91 | 91,7 |
| **11*** | 50 | 100 | 4 | 40 | 2 | 89 | 95,1 |
| **12*** | 50 | 100 | 4 | 40 | 2 | 93 | 90,4 |
| **13*** | 50 | 100 | 4 | 40 | 2 | 97 | 73,9 |
| **14*** | 50 | 100 | 4 | 40 | 2 | 91 | 89,6 |
| **15*** | 50 | 100 | 4 | 40 | 2 | 89 | 95,9 |
| **A** | 50 | 100 | 4 | 40 | 2 | 89 | 83 |

Wie der Tabelle 1 zu entnehmen ist, zeichnen sich die Liganden in der Hydroformylierung von terminalen Olefinen, hier konkreter 1-Octen, durch sehr gute Ausbeuten aus. Alle erfindungsgemäßen Verbindungen haben eine Ausbeute, welche mindestens so gut ist, wie die des Vergleichsliganden **A.**

Die Verbindungen **11** und **15**, welche die gleiche Ausbeute wie der Vergleichsligand **A** aufweisen, 89 %, haben beide aber eine deutlich bessere Selektivität.

**Tabelle 2: 2-Penten**

| **Ligand** | **p (bar)** | **T (°C)** | **t (h)** | **[Rh] (ppm)** | **L/Rh** | **Ausb. (%)** | **Sel. (%)** |
|---|---|---|---|---|---|---|---|
| **1*** | 20 | 120 | 4 | 100 | 2 | 83 | 67,3 |
| **2*** | 20 | 120 | 4 | 100 | 2 | 74 | 70,1 |
| **3*** | 20 | 120 | 4 | 100 | 2 | 93 | 57,0 |
| **4*** | 20 | 120 | 4 | 100 | 2 | 69 | 67,9 |
| **5*** | 20 | 120 | 4 | 100 | 2 | 98 | 53,1 |
| **6*** | 20 | 120 | 4 | 100 | 2 | 89 | 57,6 |
| **7*** | 20 | 120 | 4 | 100 | 2 | 68 | 56,9 |
| **8*** | 20 | 120 | 4 | 100 | 2 | 83 | 59,4 |
| **9*** | 20 | 120 | 4 | 100 | 2 | 75 | 68,4 |
| **10*** | 20 | 120 | 4 | 100 | 2 | 90 | 63,0 |
| **11*** | 20 | 120 | 4 | 100 | 2 | 47 | 79,5 |
| **12*** | 20 | 120 | 4 | 100 | 2 | 87 | 59,1 |
| **13*** | 20 | 120 | 4 | 100 | 2 | 72 | 72,5 |
| **14*** | 20 | 120 | 4 | 100 | 2 | 95 | 58,4 |
| **15*** | 20 | 120 | 4 | 100 | 2 | 93 | 75,7 |
| **B** | 20 | 120 | 4 | 96 | 2 | 14 | 99 |

Mit dem Vergleichsliganden **B** konnte für 2-Penten zwar eine sehr gute Selektivität, 99 %, erzielt werden, jedoch ist die Ausbeute mit 14 % so gering, dass der Einsatz eines solchen Ligandens für ein großtechnisches Verfahren nur wenig interessant ist. Die Raum-Zeit-Ausbeuten mit diesem Liganden sind so schlecht, sodass dies aus ökonomischer Sicht gegen den Einsatz des Vergleichsliganden **B** spricht.

Die erfindungsgemäßen Verbindungen weißen alle eine annehmbare bis sehr gute Ausbeute in Kombination mit einer guten Selektivität auf.

Wie die Versuchsergebnisse zeigen, wird die gestellte Aufgabe durch die erfindungsgemäßen Verbindungen gelöst.

Es ist somit erstmalig gelungen, Bisphosphite zu generieren, die einen unsymmetrischen Biaryl-Zentral-Baustein, also ein unsymmetrisches Rückgrat, enthalten und gute bis sehr gute Hydroformylierungseigenschaften aufweisen. Dies konnte mit einer Vielzahl von Beispielen belegt werden. Solche konkreten Strukturen und derartige Liganden waren bis dato gänzlich unbekannt und nicht zugänglich.

Diese Bisphosphite weisen eine neuartige Asymmetrie auf. Das besondere hierbei ist die Asymmetrie innerhalb des Biaryl-Zentral-Bausteins, die zu unsymmetrischen Bisphosphiten führt. Diese unsymmetrischen Bisphosphite sind strukturell somit gänzlich von denen im Stand der Technik beschriebenen Bisphoshiten verschieden, bei denen unsymmetrische Bisphosphit-Liganden durch eine bestimmte Anordnung von symmetrischen Biaryl-Bausteinen generiert werden, indem sich beispielsweise die beiden Flügel-Bausteine unterscheiden, die einzelnen Bausteine (Zentral-Baustein und Flügel-Bausteine) an sich aber symmetrisch sind.

## Patentansprüche

1. Verbindung, welche die allgemeine Struktur **II** aufweist: 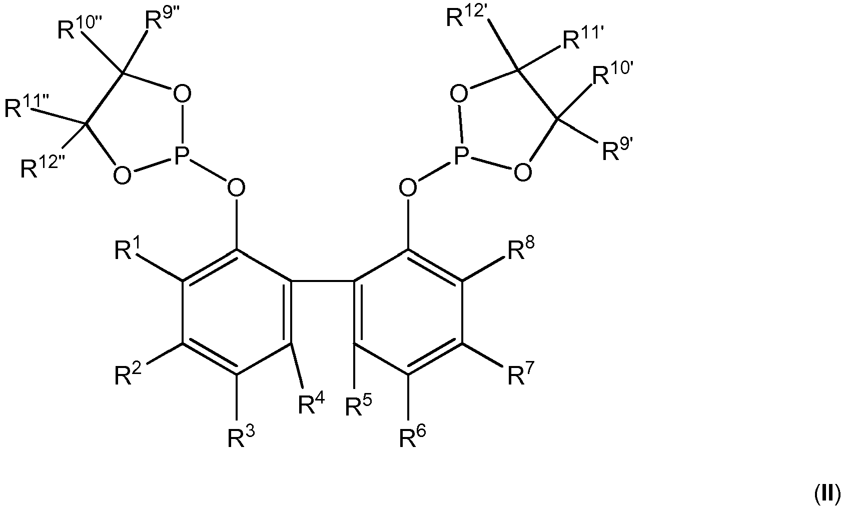 wobei
R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₆)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹', R¹⁰', R¹¹', R¹²', R⁹", R¹⁰", R¹¹", R¹²" ausgewählt sind aus:
-H, -(C₆-C₂₀)-Aryl;
und die beiden Reste mindestens eines der vier folgenden Restepaare nicht für den gleichen Rest stehen: R¹ und R⁸, R² und R⁷, R³ und R⁶, R⁴ und R⁵;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese
Substituenten sind unabhängig voneinander ausgewählt unter -H, -(C₁-C₁₂)-Alkyl, -O-(C₁-C₁₂)-Alkyl, -O-(C₆-C₂₆)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na, -NO₂, -CN, -NH₂, -N[(C₁-C₁₂)-Alkyl]₂.

2. Verbindung nach Anspruch 1,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

3. Verbindung nach einem der Ansprüche 1 oder 2,
wobei R⁹', R¹⁰', R¹¹', R¹²', R⁹", R¹⁰", R¹¹", R¹²" für -(C₆-C₂₀)-Aryl stehen.

4. Verbindung nach einem der Ansprüche 1 bis 3,
wobei R¹ und R⁸ nicht für den gleichen Rest stehen.

5. Verbindung nach einem der Ansprüche 1 bis 4,
wobei R⁹', R¹⁰', R¹¹', R¹²', R⁹", R¹⁰", R¹¹", R¹²" für den gleichen Rest stehen.

6. Komplex umfassend:
- eine Verbindung nach einem der Ansprüche 1 bis 5,
- ein Metallatom ausgewählt aus: Rh, Ru, Co, Ir.

7. Verwendung einer Verbindung nach einem der Ansprüche 1 bis 5,
zur Katalyse einer Hydroformylierungsreaktion.

8. Verfahren umfassend die Verfahrensschritte:
a) Vorlegen eines Olefins,
b) Zugabe eines Komplexes nach Anspruch 6,
oder einer Verbindung nach einem der Ansprüche 1 bis 5 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structure II: 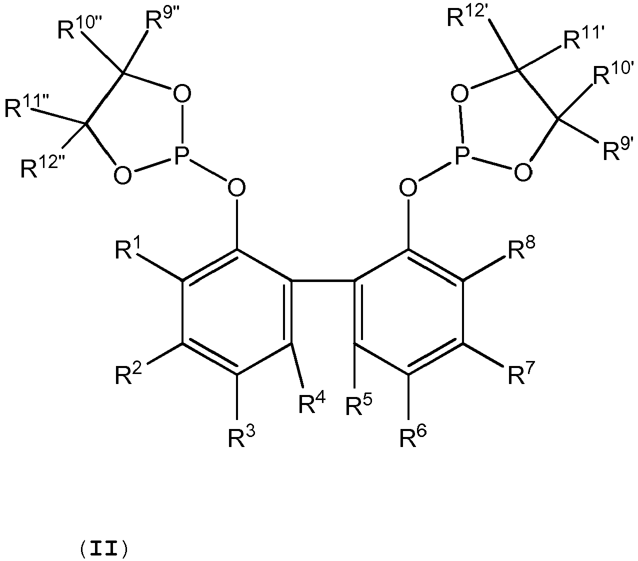 where
R¹, R², R³, R⁴, R⁵, R⁶, R⁷, R⁸ are selected from:
-H, -(C₁-C₁₂)-alkyl, -O- (C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀) -aryl, -COOH, -OH, -SO₃H, -CN, -NH₂, -N[(C₁-C₁₂)-alkyl]₂;
R⁹', R¹⁰', R¹¹', R¹²', R⁹", R¹⁰", R¹¹", R¹²" are selected from:
-H, -(C₆-C₂₀)-aryl;
and the two radicals in at least one of the four following radical pairs are not the same radical: R¹ and R⁸, R² and R⁷, R³ and R⁶, R⁴ and R⁵;
where the alkyl and aryl groups mentioned may be substituted as follows:
substituted -(C₁-C₁₂)-alkyl groups and substituted -(C₁-C₁₂)-alkoxy groups may have one or more substituents,
depending on their chain length; the substituents are each independently selected from -(C₃-C₁₂)-cycloalkyl, -(C₃-C₁₂)-heterocycloalkyl, -(C₆-C₂₀)-aryl, fluorine, chlorine, cyano, formyl, acyl or alkoxycarbonyl;
substituted -(C₆-C₂₀)-aryl groups and -(C₆-C₂₀)-aryl-(C₆-C₂₀)-aryl groups may have one or more substituents, depending on the ring size; these substituents are each independently selected from -H, -(C₁-C₁₂)-alkyl, -O-(C₁-C₁₂)-alkyl, -O-(C₆-C₂₀)-aryl, -(C₆-C₂₀)-aryl, -halogen, - COO- (C₁-C₁₂)-alkyl, -CONH- (C₁-C₁₂)-alkyl, -(C₆-C₂₀)-aryl-CON[(C₁-C₁₂)-alkyl]2, -CO-(C₁-C₁₂)-alkyl, -CO-(C₆-C₂₀)-aryl, -COOH, -OH, -SO₃H; -SO₃Na, -NO₂, -CN, -NH2, -N[(C₁-C₁₂)-alkyl]2.

2. Compound according to Claim 1,
where R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl, -S-alkyl, -S-aryl.

3. Compound according to either of Claims 1 and 2,
where R⁹', R¹⁰', R¹¹', R¹²', R⁹", R¹⁰" R¹¹", R¹²" are each -(C₆-C₂₀)-aryl.

4. Compound according to any of Claims 1 to 3,
where R¹ and R⁸ are not the same radical.

5. Compound according to any of Claims 1 to 4,
where R⁹', R¹⁰', R¹¹', R¹²', R⁹", R¹⁰", R¹¹", R¹²" are each the same radical.

6. Complex comprising:
- a compound according to any of Claims 1 to 5,
- a metal atom selected from: Rh, Ru, Co, Ir.

7. Use of a compound according to any of Claims 1 to 5 for catalysis of a hydroformylation reaction.

8. Process comprising the following process steps:
a) initially charging an olefin,
b) adding a complex according to Claim 6,
or a compound according to any of Claims 1 to 5 and a substance including a metal atom selected from: Rh, Ru, Co, Ir,
c) feeding in H₂ and CO,
d) heating the reaction mixture, with conversion of the olefin to an aldehyde.

## Revendications

1. Composé, qui présente la structure générale II : 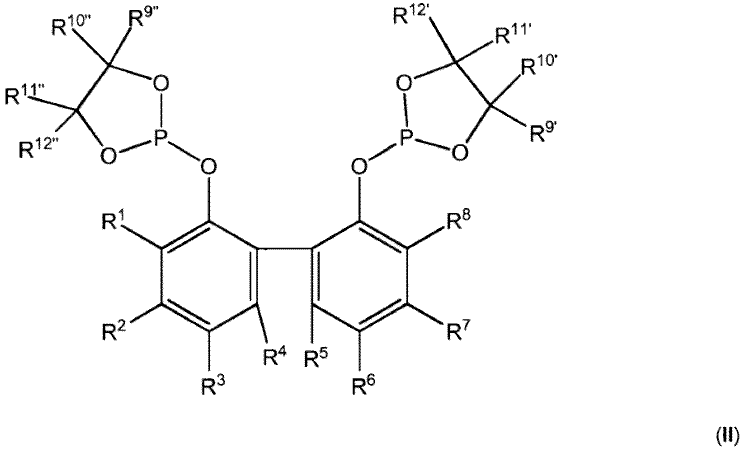 dans laquelle
R¹, R², R³, R⁴, R⁵, R⁶, R⁷, R⁸ sont choisis parmi :
-H, alkyle en (C₁-C₁₂), -O-alkyle en (C₁-C₁₂), -O-aryle en (C₆-C₂₀), aryle en (C₆-C₂₀), -S-alkyle, -S-aryle, halogène, COO-alkyle en (C₁-C₁₂), CONH-alkyle en (C₁-C₁₂), -CO-alkyle en (C₁-C₁₂), -CO-aryle en (C₆-C₂₀), -COOH, -OH, -SO₃H, -CN, -NH₂, -N[alkyle en (C₁-C₁₂)]₂ ;
R⁹', R¹⁰', R¹¹', R¹²', R⁹", R¹⁰", R¹¹", R¹²" sont choisis parmi :
-H, aryle en (C₆-C₂₀);
et les deux radicaux d'au moins une des quatre paires de radicaux suivantes ne représentent pas les mêmes radicaux : R¹ et R⁸, R² et R⁷, R³ et R⁶, R⁴ et R⁵ ;
les groupes alkyle et aryle mentionnés pouvant être substitués de la manière suivante :
les groupes alkyle en (C₁-C₁₂) substitués et les groupes alcoxy en (C₁-C₁₂) substitués peuvent comprendre un ou plusieurs substituants en fonction de leur longueur de chaîne ; les substituants sont choisis indépendamment les uns des autres parmi cycloalkyle en (C₃-C₁₂), hétérocycloalkyle en (C₃-C₁₂), aryle en (C₆-C₂₀), fluor, chlore, cyano, formyle, acyle ou alcoxycarbonyle ;
les groupes aryle en (C₆-C₂₀) substitués et les groupes aryle en (C₆-C₂₀) -aryle en (C₆-C₂₀) substitués peuvent comprendre un ou plusieurs substituants en fonction de leur taille de cycle ; ces substituants sont choisis indépendamment les uns des autres parmi -H, alkyle en (C₁-C₁₂), -O-alkyle en (C₁-C₁₂), -O-aryle en (C₆-C₂₀), aryle en (C₆-C₂₀), halogène, -COO-alkyle en (C₁-C₁₂), CONH-alkyle en (C₁-C₁₂), aryle en (C₆-C₂₀) -CON [alkyle en (C₁-C₁₂)]₂, - CO-alkyle en (C₁-C₁₂), -CO-aryle en (C₆-C₂₀), -COOH, -OH, -SO₃H, -SO₃Na, -NO₂, -CN, -NH₂, -N[alkyle en (C₁-C₁₂)]₂.

2. Composé selon la revendication 1, dans lequel R¹, R², R³, R⁴, R⁵, R⁶, R⁷, R⁸ sont choisis parmi :
-H, alkyle en (C₁-C₁₂), -O-alkyle en (C₁-C₁₂), -O-aryle en (C₆-C₂₀), -S-alkyle, -S-aryle.

3. Composé selon l'une quelconque des revendications 1 ou 2, dans lequel R^{9'}, R^{10'}, R^{11'}, R^{12'}, R^{9"}, R^{10"}, R^{11"}, R^{12"} représentent aryle en (C₆-C₂₀) .

4. Composé selon l'une quelconque des revendications 1 à 3, dans lequel R¹ et R⁸ ne représentent pas le même radical.

5. Composé selon l'une quelconque des revendications 1 à 4, dans lequel R^{9'}, R^{10'}, R^{11'}, R^{12'}, R^{9"}, R^{10"}, R^{11"}, R^{12"} représentent le même radical.

6. Complexe comprenant :
- un composé selon l'une quelconque des revendications 1 à 5,
- un atome métallique choisi parmi : Rh, Ru, Co, Ir.

7. Utilisation d'un composé l'une quelconque des revendications 1 à 5 pour la catalyse d'une réaction d'hydroformylation.

8. Procédé comprenant les étapes de procédé suivantes :
a) le chargement d'une oléfine,
b) l'ajout d'un complexe selon la revendication 6,
ou d'un composé l'une quelconque des revendications 1 à 5 et d'une substance qui comprend un atome métallique choisi parmi : Rh, Ru, Co, Ir,
c) l'ajout d'H₂ et de CO,
d) le chauffage du mélange réactionnel, l'oléfine étant transformée en un aldéhyde.
